# EUROPEAN PATENT SPECIFICATION

(11) **EP 3 960 171 B1**
(45) Date of publication and mention of the grant of the patent: **01.01.2025**
(21) Application number: 20796014.7
(22) Date of filing: 24.04.2020
(51) Int. Cl.: A61K 31/427, A61P 35/00, A61P 37/02, A61K 31/4439, A61K 31/454, A61K 31/4709, A61K 45/06

(54) **USE OF ADAM9 INHIBITOR AS IMMUNOMODULATOR**
VERWENDUNG EINES ADAM9-INHIBITORS ALS IMMUNMODULATOR
UTILISATION D'UN INHIBITEUR D'ADAM9 EN TANT QU'IMMUNOMODULATEUR

(30) Priority: 26.04.2019 US 201962839183 P
(43) Date of publication of application: 02.03.2022
(73) Proprietor: China Medical University, Taichung City 404 (TW)
(72) Inventor: SHER, Yuh-pyng, Taichung City 404, Taiwan (TW); YANG, Juan-cheng, Tainan City 702, Taiwan (TW); LIU, Jing-pei, Yuanlin City, Changhua County, Taiwan (TW); HUANG, Yu-kai, Taipei City, Taiwan (TW)
(74) Representative: D Young & Co LLP
(86) International application number: PCT/CN2020/086642
(87) International publication number: WO 2020/216319

(56) References cited:
- WO-A1-2017/153780
- WO-A1-2018/119166
- WO-A2-2004/024089
- WO-A2-2004/024089
- CN-A- 109 563 152
- TW-A- 201 917 127
- PO-LIN KUO ET AL: "Lung Cancer-derived Galectin-1 Enhances Tumorigenic Potentiation of Tumor-associated Dendritic Cells by Expressing Heparin-binding EGF-like Growth Factor", JOURNAL OF BIOLOGICAL CHEMISTRY, vol. 287, no. 13, 23 March 2012 (2012-03-23), US, pages 9753 - 9764, XP055746976, ISSN: 0021-9258, DOI: 10.1074/jbc.M111.321190
- PO-LIN KUO ET AL.: "Lung Cancer-derived Galectin-1 Enhances Tumorigenic Potentiation of Tumor-Associated Dendritic Cells by Expressing Heparin-Binding EGF-Like Growth Factor", THE JOURNAL OF BIOLOGICAL CHEMISTRY, vol. 287, no. 13, 30 January 2012 (2012-01-30), XP055746976, ISSN: 0021-9258, DOI: 10.1074/jbc.M111.321190
- SOOYEON OH ET AL.: "A Disintegrin and Metalloproteinase 9 (ADAM9) in Advanced Hepatocellular Carcinoma and Their Role as a Biomarker during Hepatocellular Carcinoma Immunotherapy", CANCERS, vol. 12, no. 3, 21 March 2020 (2020-03-21), XP055746979, ISSN: 2070-6694, DOI: 10.3390/cancers12030745

## Description

### BACKGROUND

### Technical Field

The present disclosure relates to a use of ADAM9 inhibitor. More particularly, the present disclosure relates to a use of an ADAM9 inhibitor that is a compound of Formula (I) as defined herein, or a pharmaceutically acceptable salt or stereoisomer thereof, as an immunomodulator.

### Description of Related Art

Cancer, also known as malignancy, is a state of abnormal proliferation of cells, and these proliferating cells may invade other parts of the body as a disease caused by a malfunction in the control of cell division and proliferation. The number of people suffering from cancer worldwide has a growing trend. Cancer is one of the top ten causes of death and has been ranked first among the top ten causes of death for twenty-seven consecutive years. The occurrence of cancer metastasis is the main cause of high cancer death, such as the poor prognosis of lung cancer with brain metastasis. Even if early lung cancer patients can remove the tumor by surgery, 25% will develop into distant cancer metastasis. Although common anticancer drugs can be used to inhibit cancer cell growth, they are still limited in preventing cancer recurrence. It has been recognized for many years that cancers are not controlled by the immune system, largely due to the immuno-suppressive environment of most tumors. The action of molecules in promoting metastasis can be seen as a forward-looking approach, and developing novel therapeutic strategies is urgent, such as cancer immunotherapy. For instance, WO 2017/153780 A1 discloses a combination of a CXCR4 antagonist and an immune checkpoint inhibitor, which can be used in the treatment of tumors and/or cancers.

ADAM9 (A disintegrin and metalloproteinase domain-containing protein 9) is overexpressed in tumors such as pancreatic, breast, prostate, and lung cancers; and high expression level of ADAM9 is related to the poor prognosis of cancer patients, which can be used as a predictive marker. Because ADAM9 expression can help tumor cells adapt to unfavorable environments, ADAM9 is considered to promote tumor development and is considered to be a better therapeutic target than other cancer-related metalloproteinases. ADAM9 participates in tumorigenesis due to its ability to cleave and release a number of molecules involved in cancer progression, and secretion of ADAM9 from stromal cells surrounding tumors promotes tumor development via neovascularization. For this reason, WO 2004/024089A2 discloses that inhibition of neovascularization is achieved by exposing a tissue susceptible to neovascularization to a therapeutic agent effective to inhibit ADAM 9 and/or ADAM 15 (A disintegrin and metalloproteinase domain-containing protein 15).

Previous studies demonstrated that suppression of ADAM9 expression and its downstream signaling could significantly prolong survival time of lung tumor-bearing mice. Po-Lin KUO et al. discloses that active cleavage of heparin-binding EGF-like growth factor (HB-EGF) in tumor-associated dendritic cells (TADCs) by ADAM9 and ADAM17 (A disintegrin and metalloproteinase domain-containing protein 17) is associated with increased protein kinase C δ and Lyn signaling. Enhancement of HB-EGF production in TADCs increased the proliferation, migration, and epithelial-to-mesenchymal transition abilities of lung cancer. As evident from clinical samples in lung cancer and breast cancer, we found that patients with a low expression level of ADAM9 in tumor specimens have longer survival time than that with high expression level of ADAM9. Furthermore, lack of phenotype in ADAM9 deficient mice suggests that using ADAM9 as the target protein may have good drug tolerance. For example, WO 2018/119166A1 discloses ADAM9-binding molecules, such as monospecific antibodies and bispecific, trispecific or multispecific binding molecules, including diabodies, BiTEs, and antibodies that are capable of specifically binding to ADAM9. WO 2018/119166A1 also discloses pharmaceutical compositions that contain any of such ADAM9-binding molecules, and the pharmaceutical compositions can be used in the treatment of cancer and other diseases and conditions.

### Summary

According to one aspect of the present disclosure is to provide an ADAM9 inhibitor for use in being an immunomodulator, wherein the ADAM9 inhibitor is a compound of Formula (I), or a pharmaceutically acceptable salt or stereoisomer thereof, 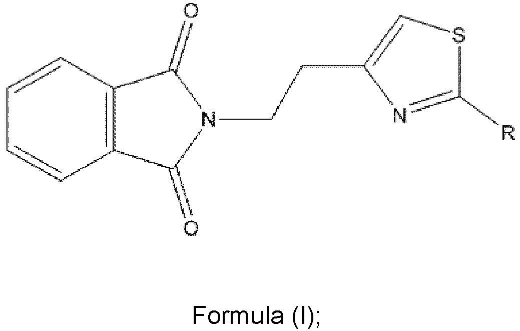 wherein R is a C₁-C₁₈ hydrocarbyl including 0-3 heteroatoms independently selected from N, S and O, or a C₁-C₁₈ hydrocarbyl including 0-3 heteroatoms independently selected from N, S and O substituted with a heteroatom selected from N, S or O; and R includes a C₅-C₁₀ organocyclic, wherein the organocyclic is an aryl including 0-3 heteroatoms independently selected from N, S and O.

According to the ADAM9 inhibitor for use, wherein the immunomodulator can be used to enhance an effectiveness of a cancer immunotherapy. Preferably, a subject of the cancer immunotherapy can be an immune-compromised patient.

According to the ADAM9 inhibitor for use, wherein the immunomodulator can be used to stimulate an infiltration of immune cells into a tumor. Preferably, the immune cells can be T cells, natural killer cells, macrophages, neutrophils, dendritic cells or suppressor cells derived from bone marrow.

According to the ADAM9 inhibitor for use, wherein the immunomodulator can be used to modify a chemokine profile of a tumor microenvironment.

According to the ADAM9 inhibitor for use, wherein the immunomodulator can be used to treat an immunotherapeutic-resistant tumor. Preferably, the immunotherapeutic-resistant tumor can be resistant to a checkpoint inhibitor, an adoptive cell transfer, a therapeutic antibody, a treatment vaccine, a cytokine, an immune cell therapy or a combination thereof, wherein the checkpoint inhibitor can be an anti-CTLA-4 antibody, an anti-PDL1 antibody or an anti-PD-1 antibody.

According to the ADAM9 inhibitor for use, wherein the immunomodulator can be formulated with a checkpoint inhibitor. Preferably, the checkpoint inhibitor can be an anti-CTLA-4 antibody, an anti-PDL1 antibody or an anti-PD-1 antibody.

According to the ADAM9 inhibitor for use, wherein R can be NHR₁, and R₁ can be 2-thiazol-4-yl-ethyl-isoindoline-1 ,3-dione.

### Brief Description of The Drawings

In order to make the above and other objectives, features, advantages and embodiments of the present disclosure more comprehensible, the description of the accompanying drawings is as follows:
Figs. 1A and 1B show the analysis results of a compound of Formula (I) inhibiting ADAM9 activity;
Figs. 2A, 2B, 2C, 2D, 2E, 2F, 2G and 2H show the analysis results of the compound of Formula (I) *in vitro;*
Fig. 3 shows the analysis results of the effect of ADAM9 knockout on tumor growth;
Figs. 4A, 4B, 4C and 4D show the RNA analysis results of ADAM9, CD4, CD8 and IFN-γ in tumor tissues after ADAM9 knockout;
Figs. 5A and 5B show the analysis results of the effect of ADAM9 knockout on lung metastatic tumor growth and immune cell infiltration;
Fig. 6 shows the analysis results of the apoptosis rate of target cells and effector cells after 24 hours of co-cultivation;
Fig. 7A shows the analysis results of the anti-tumor effect of the ADAM9 inhibitor;
Fig. 7B is a flow chart of the ADAM9 inhibitor used in the treatment of syngeneic orthotopic breast tumor animal model;
Figs. 8A and 8B show the analysis results of the effect of the ADAM9 inhibitor on immune cell infiltration;
Fig. 9 shows the analysis results of co-treatment of the ADAM9 inhibitor and the checkpoint inhibitor; and
Figs. 10A, 10B and 10C show the analysis results of the ADAM9 inhibitor stimulated tumor cells to secrete cytokines.

### Detailed Description

The following descriptions of particular embodiments and examples are provided by way of illustration and not by way of limitation. The person having ordinary skill in the art will readily recognize a variety of noncritical parameters that could be changed or modified to yield essentially similar results. The present disclosure provides myriad embodiments.

The present disclosure is to provide a use of an ADAM9 inhibitor, wherein the ADAM9 inhibitor is for use in being an immunomodulator. The immunomodulator can be used to stimulate an infiltration of immune cells into a tumor. Preferably, the immune cells can be T cells, natural killer cells, macrophages, neutrophils, dendritic cells or suppressor cells derived from bone marrow. In addition, the immunomodulator can be used to modify a chemokine profile of a tumor microenvironment or to enhance an effectiveness of a cancer immunotherapy. Preferably, a subject of the cancer immunotherapy can be an immune-compromised patient.

The present disclosure is also to provide a use of an ADAM9 inhibitor, wherein the ADAM9 inhibitor is for use in being an immunomodulator. The immunomodulator can be used to treat an immunotherapeutic-resistant tumor. Preferably, the immunotherapeutic-resistant tumor can be resistant to a checkpoint inhibitor, an adoptive cell transfer, a therapeutic antibody, a treatment vaccine, a cytokine, an immune cell therapy or a combination thereof. The checkpoint inhibitor can be a molecule used to inhibit immune checkpoint protein in the activation of immune cells; preferably, it can be an anti-CTLA-4 antibody, an anti-PDL1 antibody or an anti-PD-1 antibody.

The ADAM9 inhibitor can also be formulated with a checkpoint inhibitor to enhance the effectiveness of cancer immunotherapy. In addition, the ADAM9 inhibitor can also be used in combination with a chemotherapeutic agent and/or the checkpoint inhibitor to serve as a pharmaceutical composition for the treatment of cancer.

The ADAM9 inhibitor is a compound of Formula (I), or a pharmaceutically acceptable salt or stereoisomer thereof, 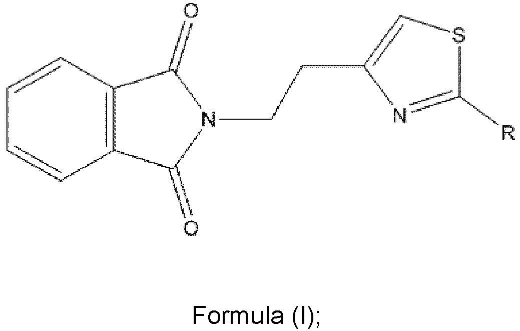 wherein R is a C₁-C₁₈ hydrocarbyl including 0-3 heteroatoms independently selected from N, S and O, or a C₁-C₁₈ hydrocarbyl including 0-3 heteroatoms independently selected from N, S and O substituted with a heteroatom selected from N, S or O; and R includes a C₅-C₁₀ organocyclic, wherein the organocyclic is aryl including 0-3 heteroatoms independently selected from N, S and O.

According to the compound of Formula (I), wherein R can be a secondary amine, amide, NHR₁, NHCOR₂, or a substituent represented by Formula (i): 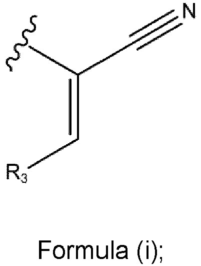 wherein R₁ can be an aryl including 0-3 heteroatoms independently selected from N, S and O, preferably R₁ can be 2-thiazol-4-yl-ethyl-isoindoline-1,3-dione; R₂ can be a phenyl or a benzoheterocyclyl including 0-3 heteroatoms independently selected from N, S and O, preferably R₂ can be benzothiophene; and R₃ can be an optionally substituted phenyl, benzoheterocyclyl or heterocyclyl including 0 to 3 heteroatoms independently selected from N, S and O.

Unless contraindicated or noted otherwise, in these descriptions and throughout this specification, the terms "a" and "an" mean one or more (that is at least one). Furthermore, genera are recited as shorthand for a recitation of all members of the genus; for example, the recitation of C₁-C₃ alkyl is shorthand for a recitation of all C₁-C₃ alkyls. For example, C₁-C₃ alkyl includes methyl, ethyl and propyl, including isomers thereof.

The following words, phrases and symbols are generally intended to have the meanings as set forth below, except to the extent that the context in which they are used indicates otherwise. The following abbreviations and terms have the indicated meanings throughout.

The term "alkenyl" in the specification refers to a hydrocarbon group selected from linear and branched hydrocarbon groups comprising at least one C=C double bond and of 2-18, or 2-12, or 2-6 carbon atoms. Examples of the alkenyl group may be selected from ethenyl or vinyl, prop-1-enyl, prop-2-enyl, 2-methylprop-1-enyl, but-1-enyl, but-2-enyl, but-3-enyl, buta-1,3-dienyl, 2-methylbuta-1,3-diene, hex-1-enyl, hex-2-enyl, hex-3-enyl, hex-4-enyl, and hexa-1,3-dienyl groups.

The term "aryl" in the specification refers to a group selected from: 5- and 6-membered carbocyclic aromatic rings, for example, phenyl; bicyclic ring system and tricyclic ring system. The bicyclic ring system can be 7-12 membered bicyclic ring systems, wherein at least one ring is carbocyclic and aromatic, which is selected from, but not limited to, naphthalene, indane, and 1,2,3,4-tetrahydroquinoline. The tricyclic ring systems can be 10-15 membered tricyclic ring systems, wherein at least one ring is carbocyclic and aromatic such as fluorene. For example, the aryl group is selected from 5- and 6-membered carbocyclic aromatic rings fused to a 5- to 7-membered cycloalkyl or heterocyclic ring optionally including at least one heteroatom selected from N, O, and S, provided that the point of attachment is at the carbocyclic aromatic ring when the carbocyclic aromatic ring is fused with a heterocyclic ring, and the point of attachment can be at the carbocyclic aromatic ring or at the cycloalkyl group when the carbocyclic aromatic ring is fused with a cycloalkyl group. Bivalent radicals formed from substituted benzene derivatives and having the free valences at ring atoms are named as substituted phenylene radicals. Bivalent radicals derived from univalent polycyclic hydrocarbon radicals whose names end in "-yl" by removal of one hydrogen atom from the carbon atom with the free valence are named by adding "-idene" to the name of the corresponding univalent radical, e.g., a naphthyl group with two points of attachment is termed naphthylidene. Aryl, however, does not encompass or overlap with heteroaryl, separately defined below. Hence, if one or more carbocyclic aromatic rings are fused with a heterocyclic aromatic ring, the resulting ring system is heteroaryl, not aryl, as defined herein.

The term "heteroaryl" in the specification refers to a group selected from: 5-to 7-membered aromatic, monocyclic rings including 1, 2, 3 or 4 heteroatoms selected from N, O, and S, with the remaining ring atoms being carbon; 8- to 12-membered bicyclic rings including 1, 2, 3 or 4 heteroatoms, selected from N, O, and S, with the remaining ring atoms being carbon and wherein at least one ring is aromatic and at least one heteroatom is present in the aromatic ring. For example, the heteroaryl group includes a 5- to 7-membered heterocyclic aromatic ring fused to a 5- to 7-membered cycloalkyl ring. For such fused, bicyclic heteroaryl ring systems wherein only one of the rings includes at least one heteroatom, the point of attachment may be at the heteroaromatic ring or at the cycloalkyl ring. When the total number of S and O atoms in the heteroaryl group exceeds 1, those heteroatoms are not adjacent to one another. In some embodiments, the total number of S and O atoms in the heteroaryl group is not more than 2. In some embodiments, the total number of S and O atoms in the aromatic heterocycle is not more than 1. Examples of the heteroaryl group include, but are not limited to, (as numbered from the linkage position assigned priority 1) pyridyl (such as 2-pyridyl, 3-pyridyl, or 4-pyridyl), cinnolinyl, pyrazinyl, 2,4-pyrimidinyl, 3,5-pyrimidinyl, 2,4-imidazolyl, imidazopyridinyl, isoxazolyl, oxazolyl, thiazolyl, isothiazolyl, thiadiazolyl, tetrazolyl, thienyl, triazinyl, benzothienyl, furyl, benzofuryl, benzoimidazolyl, indolyl, isoindolyl, indolinyl, phthalazinyl, pyrazinyl, pyridazinyl, pyrrolyl, triazolyl, quinolinyl, isoquinolinyl, pyrazolyl, pyrrolopyridinyl (such as 1H-pyrrolo[2,3-b]pyridin-5-yl), pyrazolopyridinyl (such as 1H-pyrazolo[3,4-b]pyridin-5-yl), benzoxazolyl (such as benzo[d]oxazol-6-yl), pteridinyl, purinyl, 1-oxa-2,3-diazolyl, 1-oxa-2,4-diazolyl, 1-oxa-2,5-diazolyl, 1-oxa-3,4-diazolyl, 1-thia-3,4-diazolyl, furazanyl, benzofurazanyl, benzothiophenyl, benzothiazolyl, benzoxazolyl, quinazolinyl, quinoxalinyl, naphthyridinyl, furopyridinyl, benzothiazolyl (such as benzo[d]thiazol-6-yl), indazolyl (such as 1H-indazol)-5-yl) and 5,6,7,8-tetrahydroisoquinoline.

The compounds may contain an asymmetric center and may thus exist as enantiomers. Where the compounds possess two or more asymmetric centers, they may additionally exist as diastereomers. Enantiomers and diastereomers fall within the broader class of stereoisomers. All such possible stereoisomers as substantially pure resolved enantiomers, racemic mixtures thereof, as well as mixtures of diastereomers are intended to be included. All stereoisomers of the compounds and/or pharmaceutically acceptable salts thereof are intended to be included. Unless specifically mentioned otherwise, reference to one isomer applies to any of the possible isomers. Whenever the isomeric composition is unspecified, all possible isomers are included.

"Pharmaceutically acceptable salts" in the specification include, but are not limited to, salts with inorganic acids and salts with organic acids. The salts with inorganic acids can be selected, for example, from hydrochlorates, phosphates, diphosphates, hydrobromates, sulfates, sulfinates, and nitrates. The salts with organic acids can be selected, for example, from maleates, fumarates, tartrates, succinates, citrates, lactates, methanesulfonates, p-toluenesulfonates, 2-hydroxyethylsulfonates, benzoates, salicylates, stearates, alkanoates (such as acetate), and salts with HOOC-(CH₂)ₙ-COOH, wherein n is 0 to 4. Examples of pharmaceutically acceptable cations include, but are not limited to, sodium, potassium, calcium, aluminum, lithium, and ammonium.

In addition, if a compound is obtained as an acid addition salt, the free base can be obtained by basifying a solution of the acid salt. Conversely, if the product is a free base, an addition salt (such as a pharmaceutically acceptable addition salt) may be produced by dissolving the free base in a suitable organic solvent and treating the solution with an acid, in accordance with conventional procedures for preparing acid addition salts from base compounds. Those skilled in the art will recognize various synthetic methodologies that may be used without undue experimentation to prepare non-toxic pharmaceutically acceptable addition salts.

"Treating," "treat," or "treatment" in the specification refers to the administration of at least one compound and/or at least one stereoisomer thereof, and/or at least one pharmaceutically acceptable salt thereof to a subject in recognized need thereof that has, for example, cancer.

An "effective amount" in the specification refers to an amount of at least one compound and/or at least one stereoisomer thereof, and/or at least one pharmaceutically acceptable salt thereof effective to "treat" a disease or disorder in a subject, and that will elicit, to some significant extent, the biological or medical response of a tissue, system, animal or human that is being sought, such as when administered, is sufficient to prevent development of, or alleviate to some extent, one or more of the symptoms of the condition or disorder being treated. The therapeutically effective amount will vary depending on the compound, the disease and its severity and the age, weight, etc., of the mammal to be treated.

"Cancer" in the specification refers to a physiological condition in a mammal characterized by a disorder of cell growth. A "tumor" includes one or more cancer cells. Examples of cancer include, but are not limited to, carcinoma, lymphoma, blastoma, sarcoma, and leukemia or lymphoid malignancies. More specific examples of such cancers include squamous cell carcinoma (e.g., epithelial squamous cell carcinoma), lung cancer (including small cell lung cancer, non-small cell lung cancer (NSCLC), lung adenoma, and lung squamous cell carcinoma), peritoneal cancer, hepatocellular carcinoma, gastric cancer (including gastrointestinal cancer), pancreatic cancer, glioblastoma, cervical cancer, ovarian cancer, liver cancer, bladder cancer, hepatoma, breast cancer, colon cancer, rectal cancer, colorectal cancer, endometrial cancer or uterine cancer, salivary gland cancer, kidney cancer, prostate cancer, vulvar cancer, thyroid cancer, liver cancer, anal cancer, penile cancer, and head and neck cancer.

The compound of the present disclosure, stereoisomer thereof, and pharmaceutically acceptable salt thereof may be employed alone or in combination with at least one other therapeutic agent for treatment. In some embodiments, the compound, the stereoisomer thereof, and the pharmaceutically acceptable salt thereof can be used in combination with at least one additional therapeutic agent. The at least one additional therapeutic agent can be, for example, selected from anti-hyperproliferative, anti-cancer, and chemotherapeutic agents. The compound and/or one pharmaceutically acceptable salt disclosed herein may be administered with the at least one other therapeutic agent in a single dosage form or as a separate dosage form. When administered as a separate dosage form, the at least one other therapeutic agent may be administered prior to, at the same time as, or following administration of the compound and/or one pharmaceutically acceptable salt disclosed herein.

A "chemotherapeutic agent" in the specification is a chemical compound useful in the treatment of cancer, regardless of mechanism of action. Chemotherapeutic agents include compounds used in "targeted therapy" and conventional chemotherapy. Suitable chemotherapeutic agents can be selected from: agents that induce apoptosis; polynucleotides (e.g., ribozymes); polypeptides (e.g., enzymes); drugs; biological mimetics; alkaloids; alkylating agents; antitumor antibiotics; antimetabolites; hormones; platinum compounds; monoclonal antibodies conjugated with anticancer drugs, toxins, and/or radionuclides; biological response modifiers (e.g., interferons such as IFN-α and interleukins such as IL-2); adoptive immunotherapy agents; hematopoietic growth factors; agents that induce tumor cell differentiation (e.g., all-trans-retinoic acid); gene therapy reagents; antisense therapy reagents and nucleotides; tumor vaccines; and inhibitors of angiogenesis.

Examples of chemotherapeutic agents include, but are not limited to, Erlotinib (Talecech^{®}, Genentech/OSI Pharm); Bortezomib (VELCADE^{®}, Millennium Pharm.); Fulvestrant (FASLODEX^{®}, AstraZeneca); Sunitinib (SUTENT^{®}, Pfizer); Letrozole (FEMARA^{®}, Novartis); Imatinib mesylate (GLEEVEC^{®}, Novartis); PTK787/ZK 222584 (Novartis); Oxaliplatin (Eloxatin^{®}, Sanofi); 5-FU (5-fluorouracil); Leucovorin; Rapamycin (Sirolimus, RAPAMUNE^{®}, Wyeth); Lapatinib (TYKERB^{®}, GSK572016, Glaxo Smith Kline); Lonafarnib (SCH 66336); Sorafenib (NEXAVAR^{®}, Bayer); Irinotecan (CAMPTOSAR^{®}, Pfizer) and Gefitinib (IRESSA^{®}, AstraZeneca); AG1478, AG1571 (SU 5271, Sugen); alkylating agents such as thiotepa and cyclophosphamide; alkyl sulfonates such as busulfan, improsulfan and piposulfan; aziridines such as benzodepa, carboquone, meturedopa, and uredopa; ethylenimines and methylamelamines such as altretamine, triethylenemelamine, triethylenephosphoramide, triethylenethiophosphoramide and trimethylolomelamine; acetogenins such as bullatacin and bullatacinone; camptothecin such as the synthetic analog topotecan; bryostatin; callystatin; CC-1065 and its adozelesin, carzelesin and bizelesin synthetic analogs; cryptophycins such as cryptophycin I and cryptophycin 8; dolastatin; duocarmycin and the synthetic analogs thereof (such as KW-2189 and CB1-TM1); eleutherobin; pancratistatin; sarcodictyin; spongistatin; nitrogen mustards such as chlorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, and ranimnustine; antibiotics such as the enediyne antibiotics (e.g., calicheamicin), especially calicheamicin γ1I and calicheamicin ωI1; dynemicin, such as dynemicin A; bisphosphonates, such as clodronate; esperamicin; as well as neocarzinostatin chromophore and related chromoprotein enediyne antibiotic chromophores, aclacinomycin, actinomycin, anthramycin, azaserine, bleomycin, cactinomycin, carabicin, carminomycin, carzinophilin, chromomycin, dactinomycin, daunorubicin, detorubicin, 6-diazo-5-oxo-L-norleucine, ADRIAMYCIN^{®} (doxorubicin) including morpholino-doxorubicin, cyanomorpholino-doxorubicin, 2-pyrrolino-doxorubicin and deoxydoxorubicin, epirubicin, esorubicin, idarubicin, marcellomycin, mitomycins such as mitomycin C, mycophenolic acid, nogalamycin, olivomycin, peplomycin, potfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogs such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti-adrenals such as aminoglutethimide, mitotane, trilostane; folic acid replenisher such as folinic acid; aceglatone; aldophosphamide glycoside; aminolevulinic acid; eniluracil; amsacrine; bestrabucil; bisantrene; edatraxate; defosfamide; demecolcine; diaziquone; elfornithine; elliptinium acetate; epothilone; etoglucid; gallium nitrate; hydroxyurea; lentinan; lonidamine; maytansinoids such as maytansine and ansamitocin; mitoguazone; mitoxantrone; mopidamol; nitracrine; pentostatin; phenamet; pirarubicin; losoxantrone; podophyllinic acid; 2-ethylhydrazide; procarbazine; polysaccharide complex (JHS Natural Products, Eugene, OR); razoxane; rhizoxin; sizofiran; spirogermanium; tenuazonic acid; triaziquone; 2,2',2"-trichlorotriethylamine; trichothecenes, especially T-2 toxin, verrucarin A, roridin A and anguidin; urethan; vindesine; dacarbazine; mannomustine; mitobronitol; mitolactol; pipobroman; gacytosine; arabinoside ("Ara-C"); cyclophosphamide; thiotepa; taxoids, e.g., TAXOL^{®} (paclitaxel; Bristol-Myers Squibb Oncology, Princeton, N.J.), ABRAXANE^{®} (Cremophor-free), albumin-engineered nanoparticle formulations of paclitaxel (American Pharmaceutical Partners, Schaumberg, Illinois), and TAXOTERE^{®} (doxetaxel; Rhone-Poulenc Rorer, Antony, France); chlorambucil; GEMZAR^{®} (gemcitabine); 6-thioguanine; mercaptopurine; methotrexate; platinum analogs such as cisplatin, oxaliplatin and carboplatin; vinblastine; platinum; etoposide (VP-16); ifosfamide; mitoxantrone; vincristine; NAVELBINE^{®} (vinorelbine); novantrone; teniposide; edatrexate; daunomycin; aminopterin; xeloda; ibandronate; irinotecan (Camptosar, CPT-11); topoisomerase inhibitor RFS 2000; difluoromethylornithine (DMFO); retinoids (such as retinoic acid); and pharmaceutically acceptable salts, acids and derivatives of any of the above.

The "chemotherapeutic agent" can also be selected from: (i) anti-hormonal agents that act to regulate or inhibit hormone action on tumors such as anti-estrogens and selective estrogen receptor modulators (SERM), including tamoxifen (including NOLVADEX^{®}; tamoxifen citrate), raloxifene, droloxifene, 4-hydroxytamoxifen, trioxifene, keoxifene, LY117018, onapristone, and FARESTON^{®}; (ii) aromatase inhibitors that inhibit the enzyme aromatase, which regulates estrogen production in the adrenal glands, such as 4(5)-imidazoles, aminoglutethimide, MEGASE^{®} (megestrol acetate), AROMASIN^{®} (exemestane; Pfizer), formestanie, fadrozole, RIVISOR^{®} (vorozole), FEMARA^{®} (letrozole; Novartis), and ARIMIDEX^{®} (anastrozole; AstraZeneca); (iii) anti-androgens such as flutamide, nilutamide, bicalutamide, leuprolide, and goserelin; as well as troxacitabine (1,3-dioxolane nucleoside cytosine analog); (iv) protein kinase inhibitors, such as MEK inhibitor (WO2007/044515); (v) lipid kinase inhibitors; (vi) antisense oligonucleotides, especially those which inhibit expression of genes in signaling pathways implicated in aberrant cell proliferation, such as, for example, PKC-α, Raf and H-Ras; (vii) ribozymes such as VEGF expression inhibitors (e.g., ANGIOZYME^{®}) and HER2 expression inhibitors; (viii) vaccines such as gene therapy vaccines, for example, ALLOVECTIN^{®}, LEUVECTIN^{®}, and VAXID^{®}; PROLEUHN^{®} rIL-2; topoisomerase I inhibitor such as LURTOTECAN^{®} and ABARELIX^{®} rmRH; (ix) anti-angiogenic agents such as bevacizumab (AVASTIN^{®}, Genentech); and pharmaceutically acceptable salts, acids and derivatives of any of the above.

The "chemotherapeutic agent" can also be selected from therapeutic antibodies such as alemtuzumab (Campath), bevacizumab (AVASTIN^{®}, Genentech); cetuximab (ERBITUX^{®}, Imclone); panitumumab (VECTIBIX^{®}, Amgen), rituximab (RITUXAN^{®}, Genentech/Biogen Idee), pertuzumab (OMNITARG^{™}, 2C4, Genentech), trastuzumab (HERCEPTIN^{®}, Genentech), tositumomab (Bexxar, Corixia), and the antibody drug conjugate, gemtuzumab ozogamicin (MYLOTARG^{®}, Wyeth).

Humanized monoclonal antibodies with therapeutic potential as chemotherapeutic agents in combination with a subject compound and stereoisomers thereof, and pharmaceutically acceptable salt thereof may be selected from: alemtuzumab, apolizumab, aselizumab, atlizumab, bapineuzumab, bevacizumab, bivatuzumab mertansine, cantuzumab mertansine, cedelizumab, certolizumab pegol, cidfusituzumab, cidtuzumab, daclizumab, eculizumab, efalizumab, epratuzumab, erlizumab, felvizumab, fontolizumab, gemtuzumab ozogamicin, inotuzumab ozogamicin, ipilimumab, labetuzumab, lintuzumab, matuzumab, mepolizumab, motavizumab, motovizumab, natalizumab, nimotuzumab, nolovizumab, numavizumab, ocrelizumab, omalizumab, palivizumab, pascolizumab, pecfusituzumab, pectuzumab, pertuzumab, pexelizumab, ralivizumab, ranibizumab, reslivizumab, reslizumab, resyvizumab, rovelizumab, ruplizumab, sibrotuzumab, siplizumab, sontuzumab, tacatuzumab tetraxetan, tadocizumab, talizumab, tefibazumab, tocilizumab, toralizumab, trastuzumab, tucotuzumab celmoleukin, tucusituzumab, umavizumab, urtoxazumab, and visilizumab.

The following specific examples are used to further illustrate the present disclosure, in order to benefit the person having ordinary skill in the art, and can fully utilize and practice the present disclosure without excessive interpretation. These examples should not be regarded as limiting the scope of the present disclosure, but is used to illustrate how to implement the materials and methods of the present disclosure.

A novel use of an ADAM9 inhibitor is provided. The ADAM9 inhibitor is for use in being an immunomodulator, wherein the ADAM9 inhibitor is a compound of Formula (I) as defined herein. The following examples of the ADAM9 inhibitor according to the present invention illustrate that the ADAM9 inhibitor has the effects requested in the scope of the present disclosure.

### I. Synthesis and identification of the compound of Formula (I)

The derivatives with the structure represented by Formula (I) in the embodiments of the present disclosure are designed and synthesized for the structure that can inhibit the activity of ADAM9, and the molecular docking approach was used to virtually screen compounds that can fit into the catalytic site of the metalloprotease domain of ADAM9.

The structural formulas of Example 1 to Example 32 of the compound of the Formula (I) of the present disclosure are shown in Table 1 as follows.

**Table 1**

| Example | Compound | Structural formula |
|---|---|---|
| 1 | 2X-0295 | 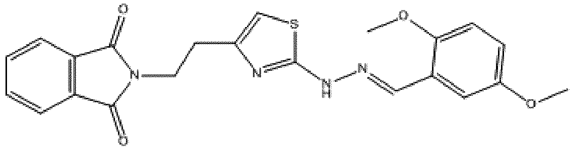 |
| 2 | 2X-0336 | 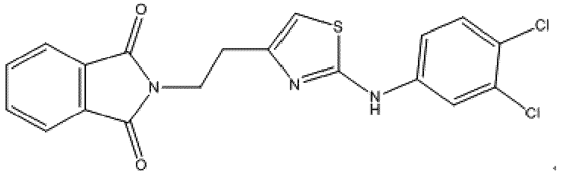 |
| 3 | 4X-0296 | 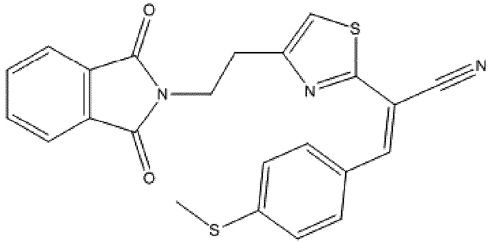 |
| 4 | 4X-0301 | 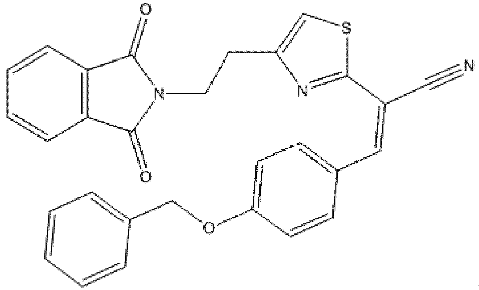 |
| 5 | 4X-0302 | 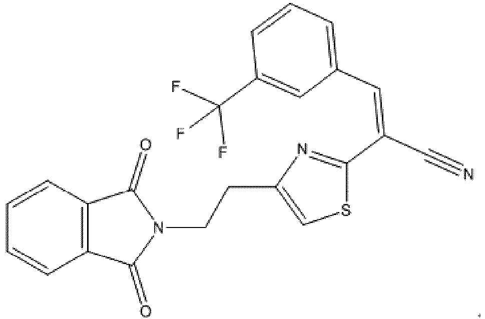 |
| 6 | 4X-0311 | 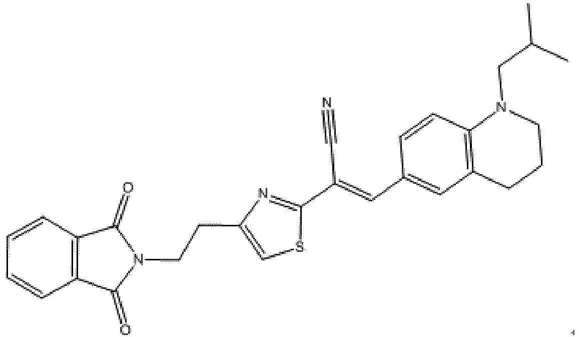 |
| 7 | 4X-0312 | 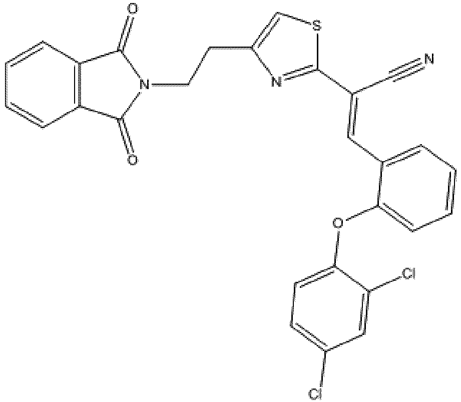 |
| 8 | 4X-0314 | 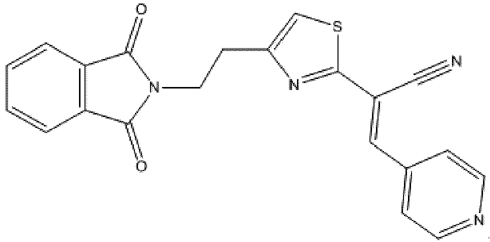 |
| 9 | 5X-0314 | 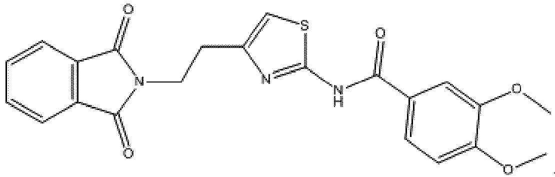 |
| 10 | 6X-0202 | 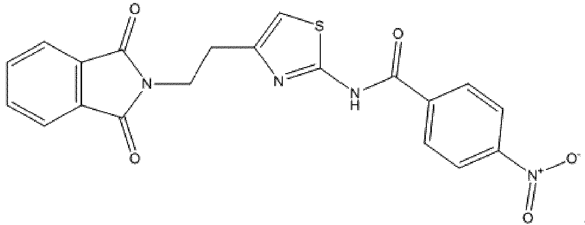 |
| 11 | SW2 | 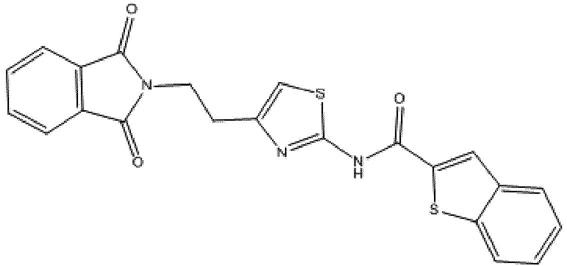 |
| 12 | 6X-0229 | 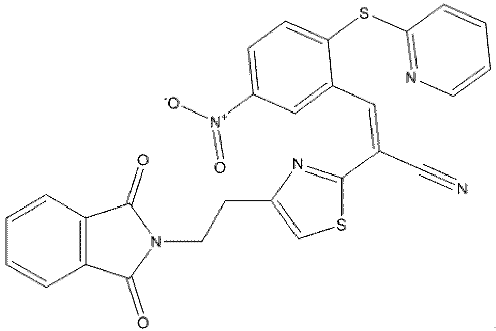 |
| 13 | 6X-0309 | 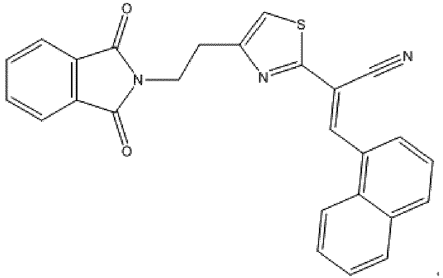 |
| 14 | 6X-0310 | 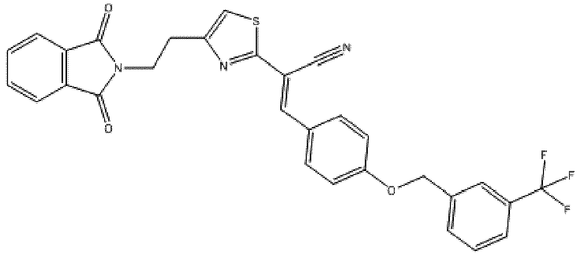 |
| 15 | 6X-0311 | 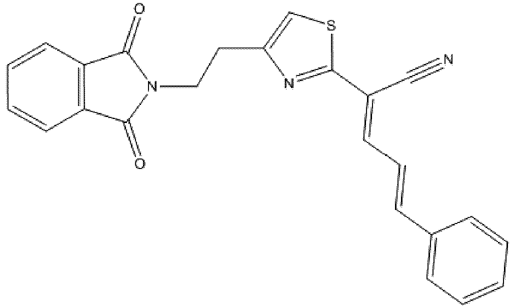 |
| 16 | 11W-0296 | 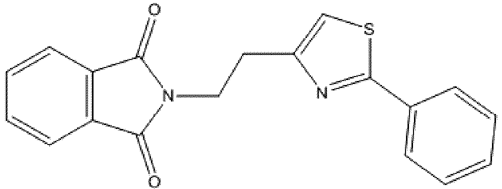 |
| 17 | 12W-0232 | 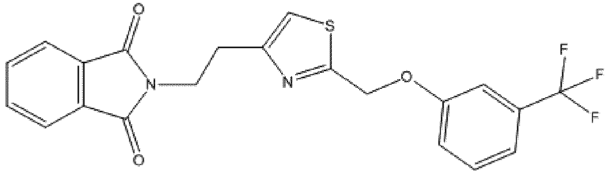 |
| 18 | 12W-0264 | 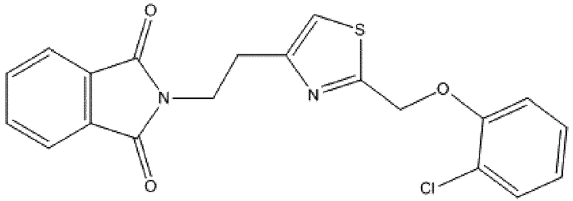 |
| 19 | 12W-0266 | 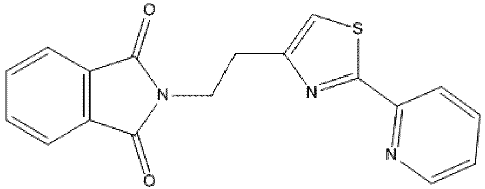 |
| 20 | SW1 | 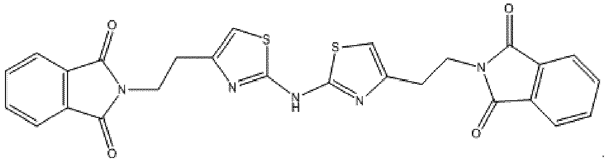 |
| 21 | 12W-0272 | 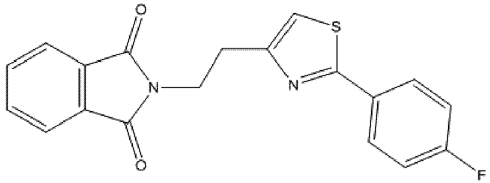 |
| 22 | 12W-0274 | 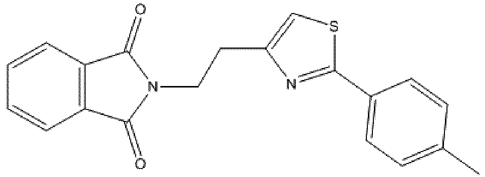 |
| 23 | 12W-0275 | 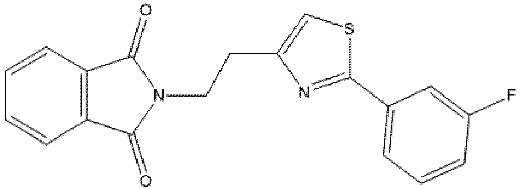 |
| 24 | 12W-0290 | 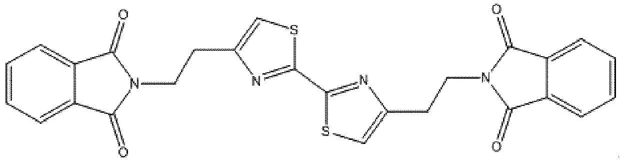 |
| 25 | 13W-0300 | 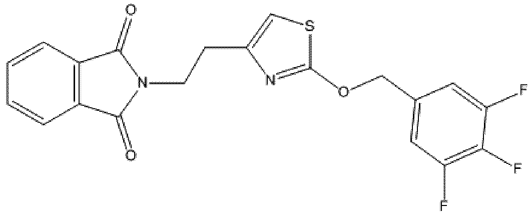 |
| 26 | 13W-0301 | 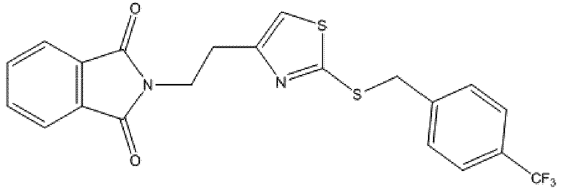 |
| 27 | 13W-0302 | 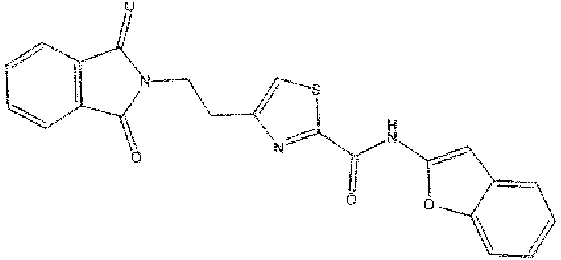 |
| 28 | 13W-0303 | 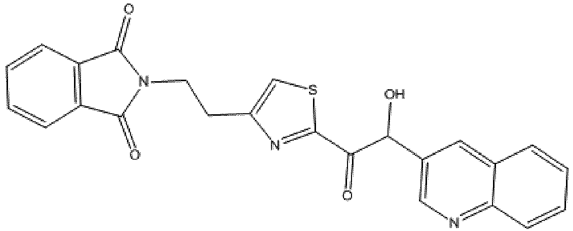 |
| 29 | 13W-0304 | 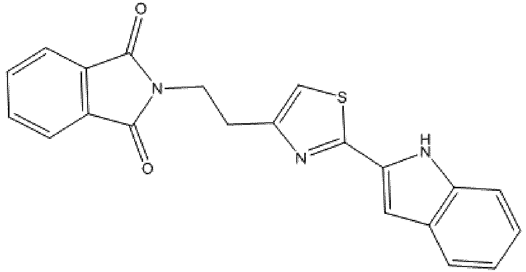 |
| 30 | 13W-0305 | 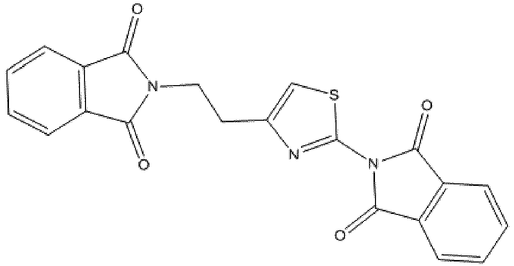 |
| 31 | 13W-0306 | 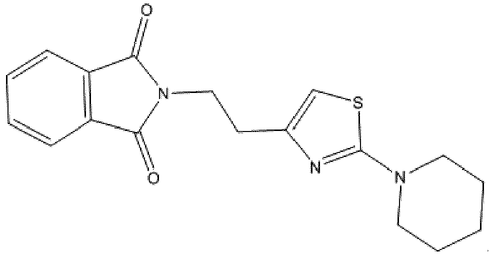 |
| 32 | 13W-0307 | 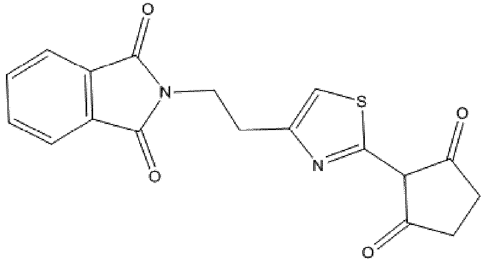 |

In the experiment, the derivatives of compound 6X-0310 were selected in Example 1 to Example 32 for further investigation. The selected compounds were the compound 4X-0296, the compound SW1, the compound 2X-0295, the compound SW2, the compound 12W-0264, and the compound 6X-0310. The compound 9R-0655, the compound MS-1176, the compound 5W-0369, and the compound 161 were also included in the experiment, which were not based on the compound 6X-0310 as the core structure.

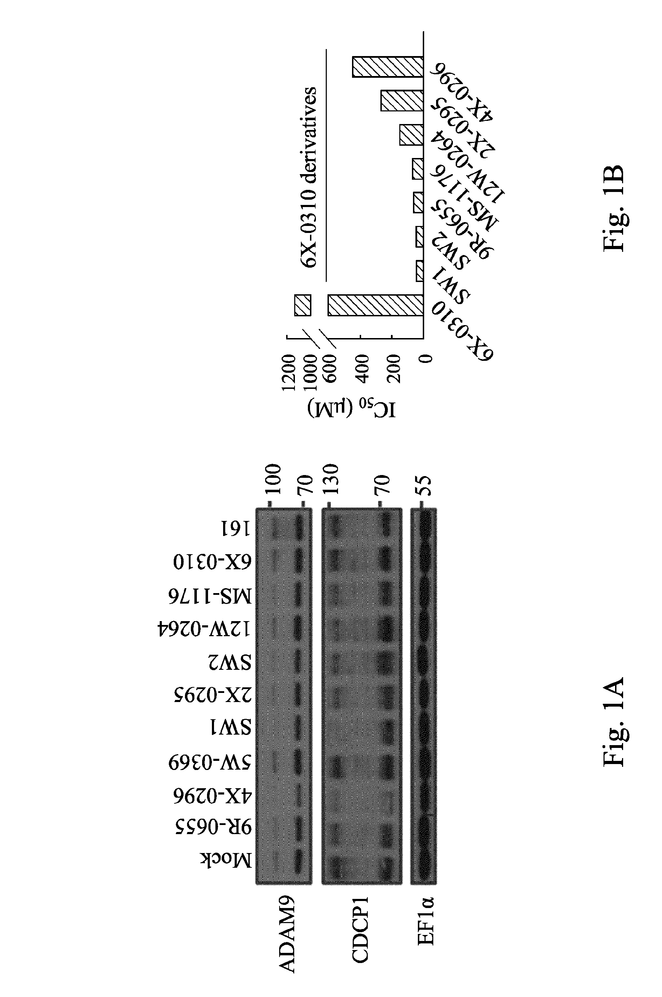

Please refer to Fig. 1A, Fig. 1B and Table 2, which show the analysis results of the compound of Formula (I) inhibiting ADAM9 activity. In Fig. 1A, Bm7 lung cancer cells were treated with the derivatives of the compound 6X-0310, and then cultured under anchorage-free culture condition. The expressions of ADAM9 and CDCP1 in Bm7 lung cancer cells were detected by Western blot, in which EF1α is an internal control, and mock represents Bm7 lung cancer cells without treatment. The results of Fig. 1A show that the compound 4X-0296, the compound SW1, the compound 2X-0295, the compound SW2, the compound 12W-0264, and the compound 6X-0310 can reduce the expression of CDCP1. In the experiment, the compounds of Formula (I) of Example 1 to Example 32 were further performed cytotoxicity assay to measure the IC₅₀ value of the compounds of Formula (I) of the present disclosure in lung cancer cell lines. Please refer to Fig. 1B and Table 2 for the experimental results, Fig. 1B shows the cytotoxicity assay results in Bm7 lung cancer cells treated with the compound 9R-0655, the compound 4X-0296, the compound SW1, the compound 2X-0295, the compound SW2, the compound 12W-0264, the compound MS-1176 and the compound 6X-0310. Table 2 shows the cytotoxicity assay results of Example 1 to Example 32 in different tumor cell lines, in which "+" represents that the compound has the effect of inhibiting tumor cell growth, and the number is the IC₅₀ value (unit is µM).

**Table 2**

| | 293 | HNOF | A549 | H1299 | TC1 | Bm7brm x2 | Bm7brm x2-1st | Bm7brmx2 -2nd | 231-brm | PE10 WBC |
|---|---|---|---|---|---|---|---|---|---|---|
| 2X-0295 | + 77.38 | + 78.36 | + 1018.87 | + 72.04 | + 78.98 | + 75.22 | + 53.21 | + 267.05 | | |
| 2X-0336 | + 58.70 | + 80.00 | + 127.89 | + 100.63 | + 55.94 | + 117.20 | + 37.64 | + 62.96 | | |
| 4X-0296 | + 79.21 | + 96.15 | + 166.67 | + 94.52 | + 73.56 | + 92.04 | + 39.32 | + 447.42 | | |
| 4X-0301 | + 49.47 | + 77.57 | + 160.69 | + 102.03 | + 71.93 | + 128.12 | + 61.24 | + 310.06 | | |
| 4X-0302 | + 55.71 | + 151.73 | + 115.86 | + 189.77 | + 125.47 | + 852.37 | + 519.79 | + 433.56 | | |
| 4X-0311 | + 62.92 | + 80.74 | + 237.69 | + >125 | + 345.26 | + 60.59 | + 34.36 | + 86.62 | | |
| 4X-0312 | + 134.45 | + 262.41 | + 644.23 | + 120.73 | + 135.60 | + 300.56 | + 63.42 | + 491.31 | | |
| 4X-0314 | + 268.51 | + 152.70 | + 526.97 | + 38427.78 | + 175.55 | + 201.75 | + 1421.11 | + 15700.42 | | |
| 5X-0314 | + 49.33 | + 85.71 | + 183.89 | + 115.76 | + 202.40 | + 66.50 | + 50.99 | + 90.64 | | |
| 6X-0202 | + 47.61 | + 78.29 | + 765.74 | + 335.39 | + 124.55 | + >125 | + 68.64 | + 1272.20 | | |
| SW2 | + 15.02 | + 61.87 | + 457.79 | + 32.05 | + 64.15 | + 52.73 | + 44.31 | + 45.06 | + 7.55 | + 76.45 |
| 6X-0229 | + 132.66 | + 145.39 | + 108.81 | + 170.73 | + 166.67 | + 374.17 | + 56.18 | + 113.26 | | |
| 6X-0309 | + 82.17 | + 113.86 | + >125 | + 93.84 | + 89.41 | + 89.77 | + 627.84 | + 384.92 | | |
| 6X-0310 | + 58.85 | + 141.25 | + 199.93 | + 116.04 | + 110.71 | + 175.76 | + 54.55 | + 250.40 | | |
| 6X-0311 | + 231.10 | + 410.98 | + 210.68 | + 505.32 | + >125 | + >125 | + >50 | + 590.29 | | |
| 11W-0296 | + 62.56 | + 92.01 | + 420.83 | + 233.84 | + 259.30 | + 1256.68 | + 175.41 | + 147.46 | | |
| 12W-0232 | + 73.60 | + 103.44 | + 184.10 | + 119.13 | + 99.20 | + 138.49 | + >50 | + 77.67 | | |
| 12W-0264 | + 59.74 | + >125 | + 161.74 | + 137.86 | + 88.87 | + 99.22 | + 40.94 | + 151.38 | | |
| 12W-0266 | + 121.60 | + 183.83 | + 190.18 | + 253.05 | + 169.36 | + 429.34 | + 88.79 | + 133.70 | | |
| SW1 | + 81.26 | + 97.77 | + 434.33 | + 76.93 | + 80.40 | + 71.56 | + 40.38 | + 43.49 | + 4.50 | + 14509 |
| 12W-0272 | + 133.50 | + 600.30 | + 177.34 | + 844.87 | + 822.56 | + 390.77 | + 129.99 | + 264.35 | | |
| 12W-0274 | + 21.84 | + 125.10 | + >125 | + 203.39 | + 161.35 | + 913.41 | + 80.59 | + 471.79 | | |
| 12W-0275 | + 109.25 | + 129.23 | + 198.68 | + 156.48 | + 103.30 | + 212.07 | + 97.03 | + 440.96 | | |
| 12W-0290 | + 69.39 | + 147.01 | + >125 | + 159.13 | + 398.96 | + 845.10 | + 84.18 | + 3976.53 | | |
| 13W-0300 | + | + | + | + | + | + | + | + | | |
| 13W-0301 | + | + | + | + | + | + | + | + | | |
| 13W-0302 | + | + | + | + | + | + | + | + | | |
| 13W-0303 | + | + | + | + | + | + | + | + | | |
| 13W-0304 | + | + | + | + | + | + | + | + | | |
| 13W-0305 | + | + | + | + | + | + | + | + | | |
| 13W-0306 | + | + | + | + | + | + | + | + | | |
| 13W-0307 | + | + | + | + | + | + | + | + | | |

The results in Fig. 1B and Table 2 show that Examples 1 to 32 have toxic effects on kidney cancer cells (293), breast cancer cells (231-brm) and different lung cancer cells (A549, H1299, TC1, Bm7brmx2, Bm7brmx2-1st, Bm7brmx2-2nd and PE10 WBC). Compared with the core structure-the compound 6X-0310, the compound SW1, the compound SW2, the compound 12W-0264, the compound 2X-0295 and the compound 4X-0296 have stronger tumor cell cytotoxicity, especially the compound SW1 and the compound SW2.

In the experiment, the Ki (inhibitory constant) of Matrix metalloproteinase (MMP) inhibitor-CGS27023A (Novartis), and the compound SW1 and the compound SW2 were calculated using Autodock v.4.2 to evaluate whether they are potential ADAM9 inhibitors. Please refer to Table 3 for evaluation results.

**Table 3**

| Compound | Binding Energy (kcal) | Ki (Inhibitory constant) |
|---|---|---|
| CGS27023A | -6.64 | 13470 nM |
| SW1 | -9.49 | 100 nM |
| SW2 | -9.49 | 100 nM |

The results in Table 3 show that the Ki values of the compound SW1 and the compound SW2 are lower than the MMP inhibitor-CGS27023A in support of our contention that a lower concentration of the ADAM9 inhibitor provides the same inhibitory effect as a higher concentration of the CGS27023A.

### II. The inhibitory effect of the compound of Formula (I) on ADAM9 activity

To measure the efficacy of the compound of Formula (I) in suppression of ADAM9 enzymatic activity, an ELISA system was established using recombinant human ADAM9 and a fluorescent peptide substrate (R&D system) in the experiment. Broad-spectrum MMP inhibitor-BB-94 was included as a positive control. The compounds of Formula (I) used in this experiment were the compound SW1 and the compound SW2.

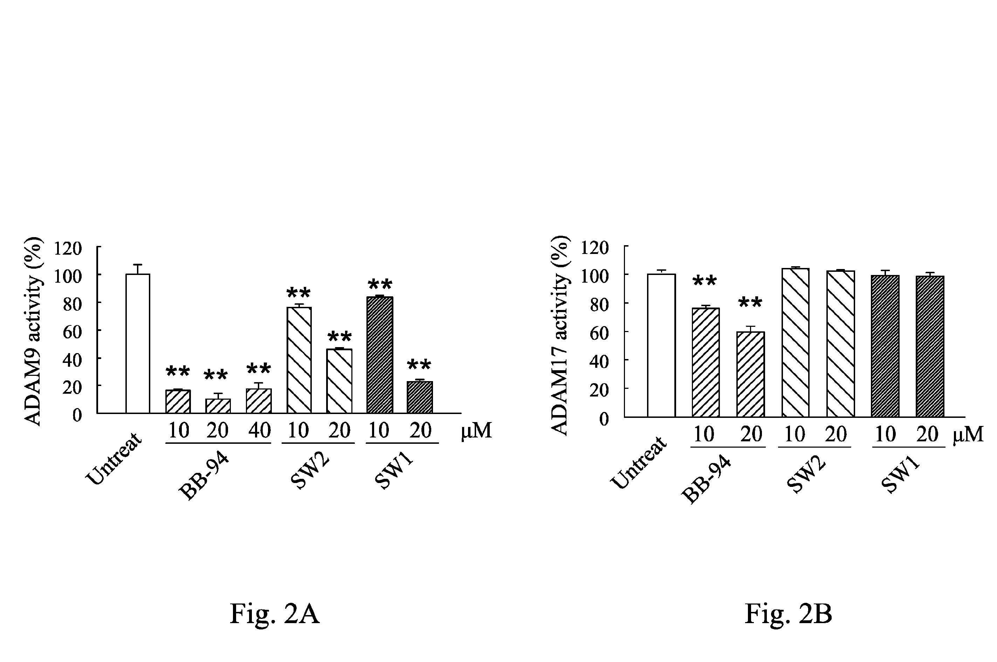

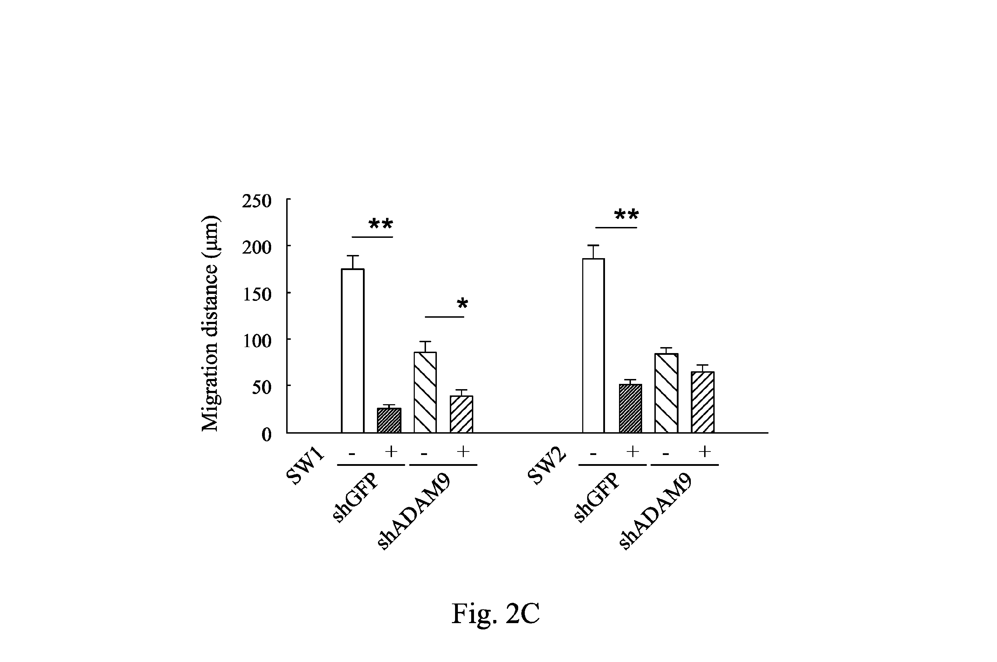

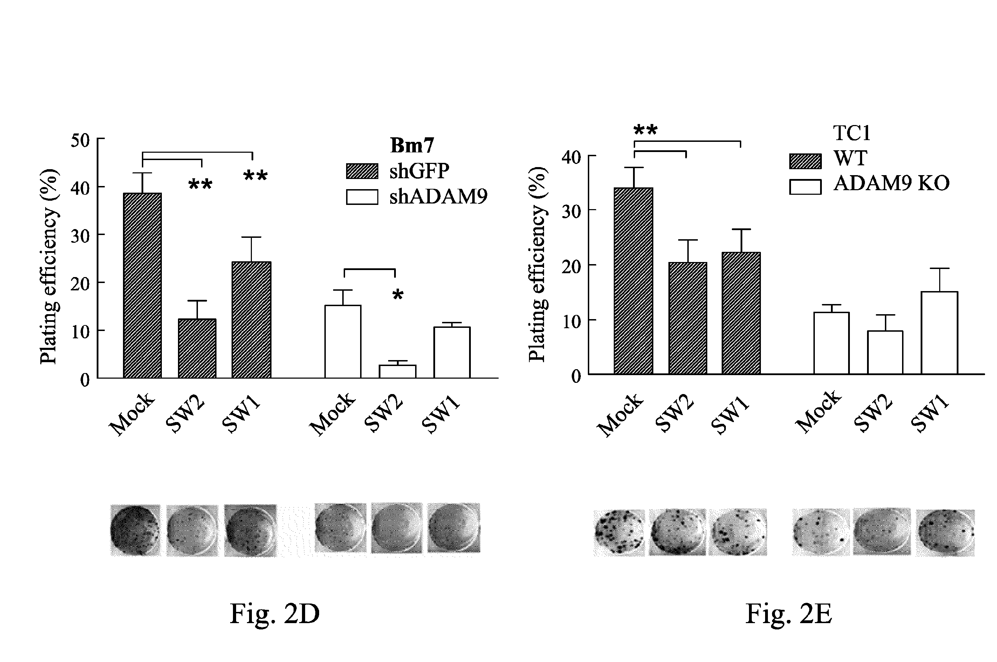

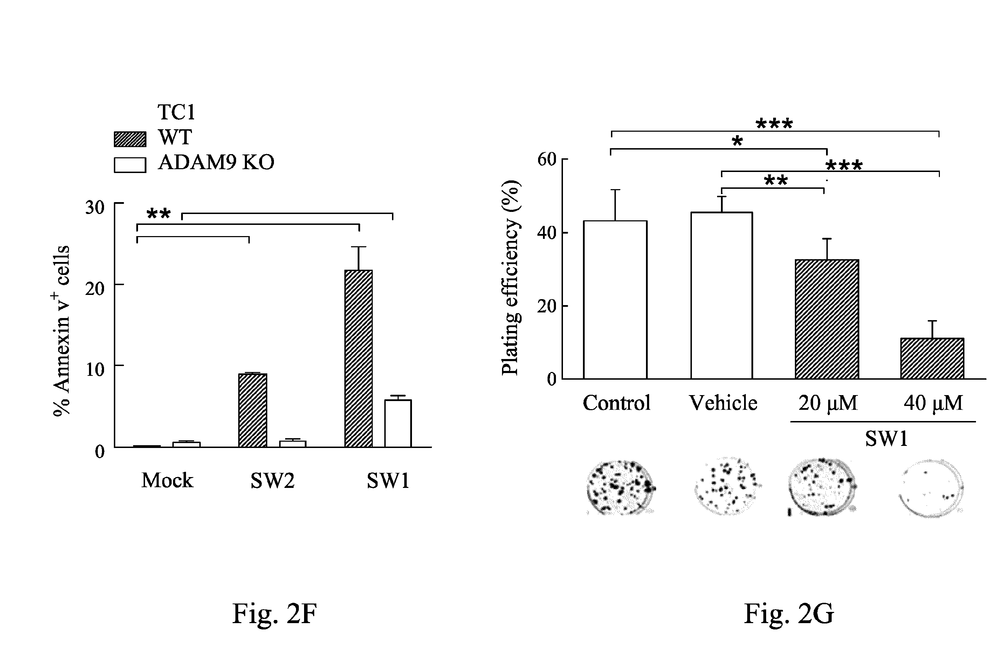

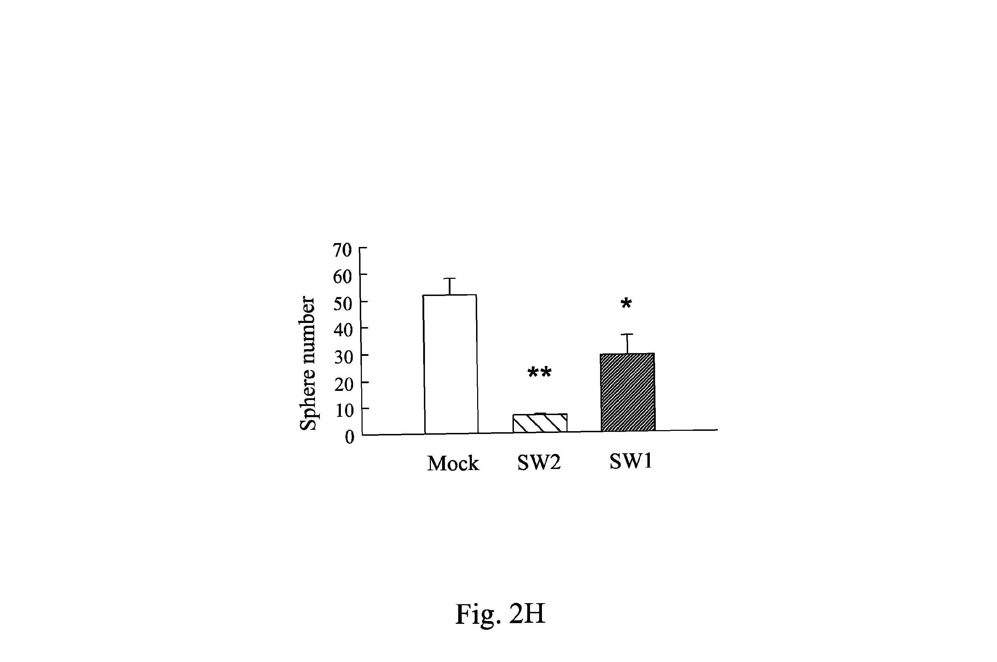

Figs. 2A, 2B, 2C, 2D, 2E, 2F, 2G and 2H show the characteristics analysis results of the compound SW1 and the compound SW2 *in vitro* of the embodiment of the present disclosure.

Fig. 2A shows the analysis of the inhibition of ADAM9 activity with different concentrations of the compound SW1 and the compound SW2. Fig. 2B shows the analysis of the inhibition of ADAM17 activity with different concentrations of the compound SW1 and the compound SW2. The results in Figs. 2A and 2B show that the compound SW1 and the compound SW2 decrease ADAM9 activity to a similar extent in a dose-dependent manner. However, the compound SW1 and the compound SW2 have no inhibitory effects in ADAM17.

To further evaluate the specificity of the compound SW1 and the compound SW2, control Bm7 lung cancer cells (treated with shGFP) and ADAM9 knockout (KO) Bm7 lung cancer cells (treated with shADAM9) were treated with 25 µM of the compound SW1 or the compound SW2, respectively, and then performed a migration inhibition assay. Please refer to Fig. 2C, the results show that the treatment of the compound SW1 or the compound SW2 can significantly reduce the migration ability of control Bm7 lung cancer cells.

To ensure the compound SW1 and the compound SW2 in blocking anoikis-resistance of cancer cells, the IC₅₀ value (unit is µM) of the compound SW1 and the compound SW2 for anoikis (anchorage-free induced apoptosis) in lung cancer brain metastatic cell line-Bm7brmx2, breast cancer brain metastatic cell line-MDA-231brm and leukocytes in anchorage-free culture conditions were determined. Please refer to Table 4, the results show that the compound SW1 and the compound SW2 can induce cell death under anchorage-free culture conditions, and provide good therapeutic effects in the breast cancer brain metastasis cell line-MDA-231brm.

**Table 4**

| Cell type | Cell line | SW2 | SW1 |
|---|---|---|---|
| Lung cancer brain metastatic | Bm7brmx2 | 27 | 20 |
| Breast cancer brain metastatic | MDA-231brm | 7.5 | 4.5 |
| Leukocytes | | 76 | >200 |

To further test the planting efficiency of tumor cells treated with the compound SW1 and the compound SW2, in the experiment, control Bm7 lung cancer cells (treated with shGFP) and ADAM9 KO Bm7 lung cancer cells (treated with shADAM9) were treated with 20 µM of the compound SW1 or the compound SW2 respectively, and TC1-wild-type (WT) lung cancer cells and TC1-ADAM9 KO lung cancer cells were treated with 10 µM of the compound SW1 or the compound SW2 respectively. Then colony-forming assay was used to confirm the planting efficiency of each test group. Please refer to Figs. 2D and 2E, wherein mock represents the tumor cell lines without treatment of the compound SW1 or the compound SW2. Representative images of colony-forming assay are shown at the bottom, and data are means ± SD of 6 separate wells. The results show that the treatment of the compound SW1 or the compound SW2 can greatly reduce the planting efficiency of control Bm7 lung cancer cells and TC1-WT lung cancer cells, and indicate that the compound SW1 and the compound SW2 can reduce the growth of control Bm7 lung cancer cells and TC1-WT lung cancer cells.

In the experiment, TC1-WT lung cancer cells and TC1-ADAM9 KO lung cancer cells were further treated with the compound SW1 or the compound SW2 for 24 hours, and then stained with Annexin V and PI to detect cell apoptosis. Please refer to Fig. 2F, where the percentage of Annexin V⁺ cells (apoptotic cells) is the statistical result from three independent experiments, with mean ± SD. The results in Fig. 2F show that compared with TC1-ADAM9 KO lung cancer cells, the compound SW1 and the compound SW2 can significantly reduce the growth of control lung cancer cells and induce cell apoptosis.

In addition, the IC₅₀ values (unit is µM) of the compound SW1 and the compound SW2 on pancreatic cancer cells were measured in the experiment. Please refer to Fig. 2G and Table 5, the results show that the compound SW1 and the compound SW2 of the present disclosure have cytotoxicity on different pancreatic cancer cells.

**Table 5**

| Pancreatic cancer cells | SW2 | SW1 |
|---|---|---|
| Panc-1 | 34.2 | 424 |
| MiaPaCa | 8.5 | - |
| PC-080 | 89.6 | 446 |
| Pan18-GFP-Luc | 112 | 0.3 |
| Su86-86-GFP-Luc | 66.8 | 59.2 |
| HPAC-GFP-Luc | 29.3 | 126.5 |

In the experiment, the Bm7 lung cancer cells were treated with 25 µM of the compound SW1 or the compound SW2 for 12 hours, and the number of tumor spheres formed was determined. Please refer to Fig. 2H, where mock represents Bm7 lung cancer cells without treatment. The results show that the treatment of the compound SW1 and the compound SW2 can greatly reduce the number of tumor spheres (* represents p < 0.05, ** represents p < 0.01).

### III. The effect of the ADAM9 inhibitor in cancer immunotherapy

To verify the effect of ADAM9 on the immune response to tumor progression, TC1-WT lung cancer cells (n = 10) and TC1-ADAM9 KO lung cancer cells (n = 10) were injected subcutaneously into C57BL/6 mice with normal immunity to establish a subcutaneous lung tumor mouse model. On Day 22 after cell injection, the mice in TC1-WT group and TC1-ADAM9 KO group were sacrificed, and their tumor tissues were taken out, the volume of the tumor tissue was measured, and three separate tumor samples in each group were used RT-qPCR to analyze the RNA expression of ADAM9, CD4, CD8 and IFN-γ in tumor tissues.

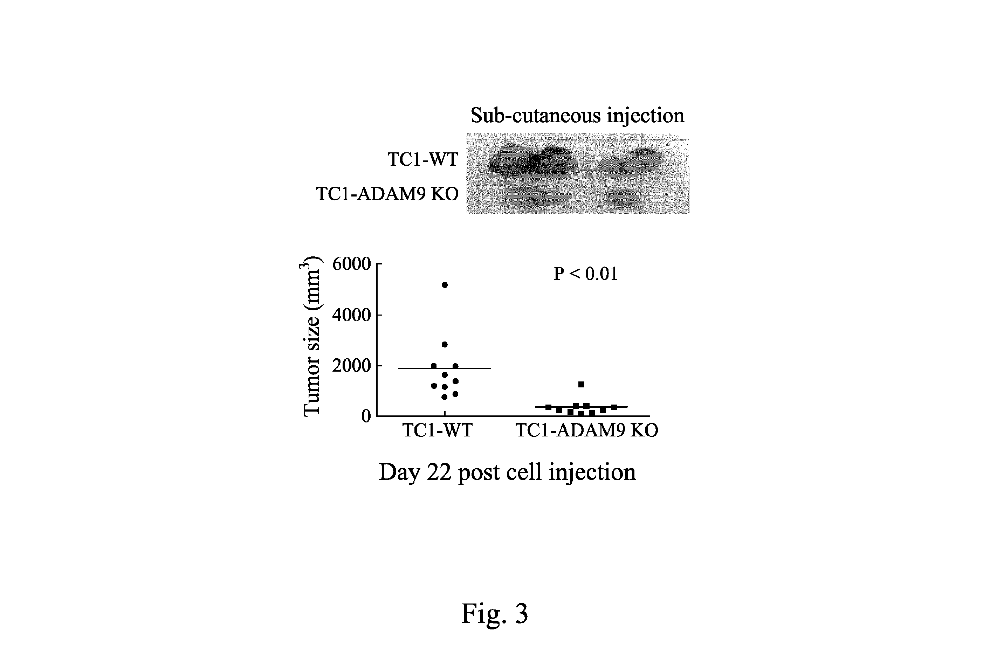

Please refer to Fig. 3 for the analysis results of the effect of ADAM9 knockout on tumor growth, which is a photo diagram and statistical result diagram of the tumor tissue size of mice in TC1-WT group and TC1-ADAM9 KO group. In Fig. 3, TC1-ADAM9 KO lung cancer cells reduce tumor size and metastatic nodules in the subcutaneous lung tumor mouse model.

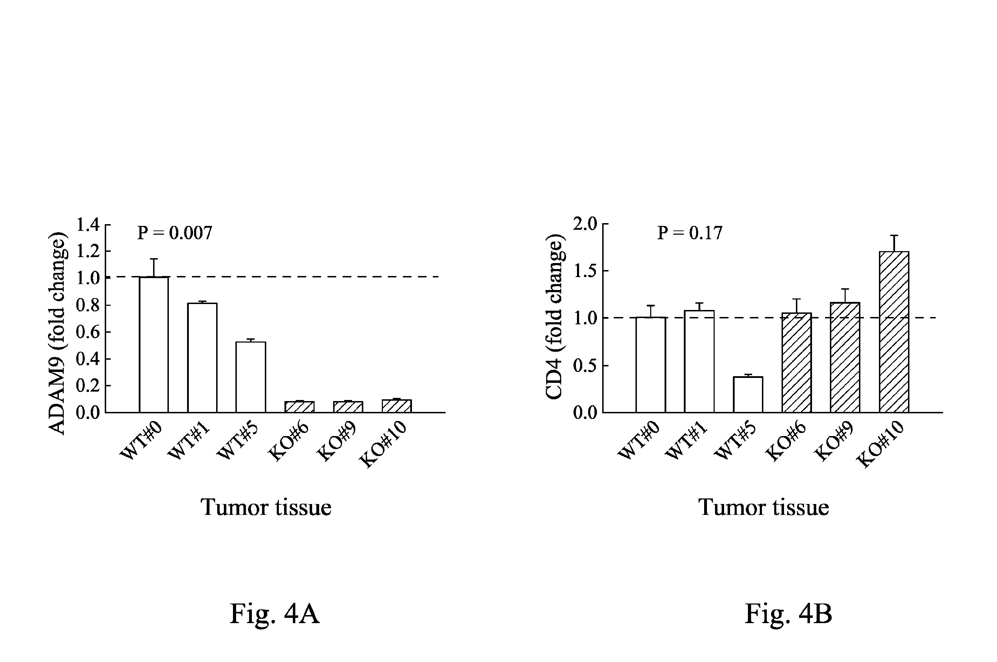

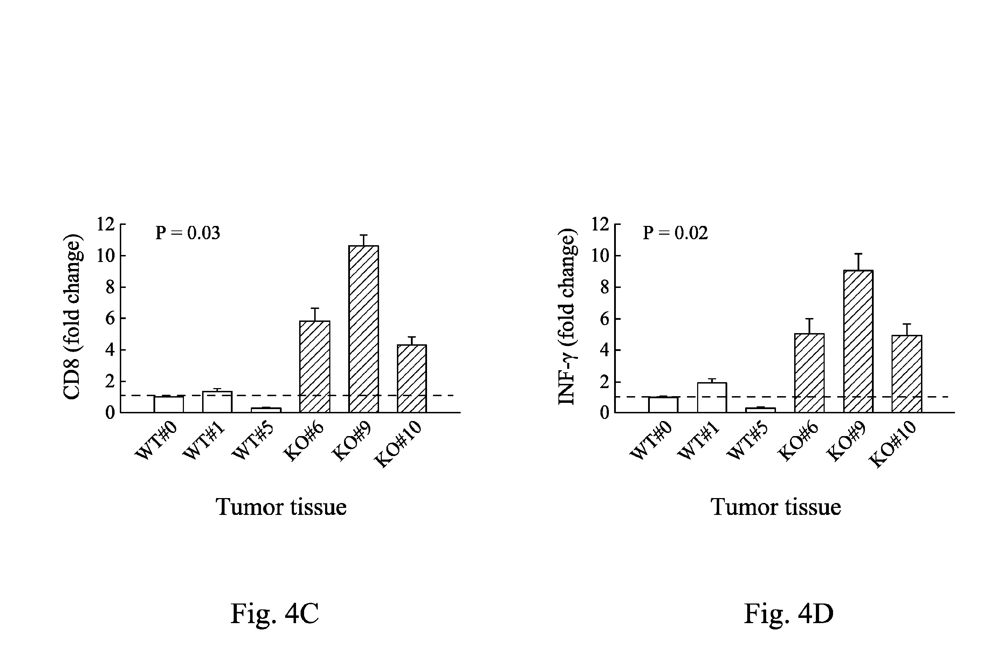

Please refer to Figs. 4A, 4B, 4C and 4D, which show the RNA analysis results of ADAM9, CD4, CD8 and IFN-γ in tumor tissues after ADAM9 knockout, wherein WT represents mice in TC1-WT group, and KO represents mice in TC1-ADAM9 KO group. The results show that RNA of CD8 and IFN-γ were overexpressed in tumors of mice in TC1-ADAM9 KO group. Since neither CD8 nor IFN-γ were detected in the cultured TC1-ADAM9 KO lung cancer cells but high levels of them were found in tumors of mice in TC1-ADAM9 KO group, these results indicate that ADAM9 knockout may increase the infiltration of CD8⁺ T cells and the expression of IFN-γ in tumor tissues, and can induce an immune response that suppresses TC1 tumor progression.

In order to further confirm whether ADAM9 gene knockout also affects the immune response of lung metastatic tumors, TC1-WT lung cancer cells (n = 5) and TC1-ADAM9 KO lung cancer cells (n = 5) were injected into C57BL/6 mice with normal immunity through the tail vein to establish a lung metastasis model. On Day 28 after the cell injection, the mice in TC1-WT group and TC1-ADAM9 KO group were sacrificed, their lung tissues were taken out, and the lung tissue sections were subjected to immunohistochemistry to analyze the immune cell profile, and count the number of immune cells in each range of the results of immunohistochemistry.

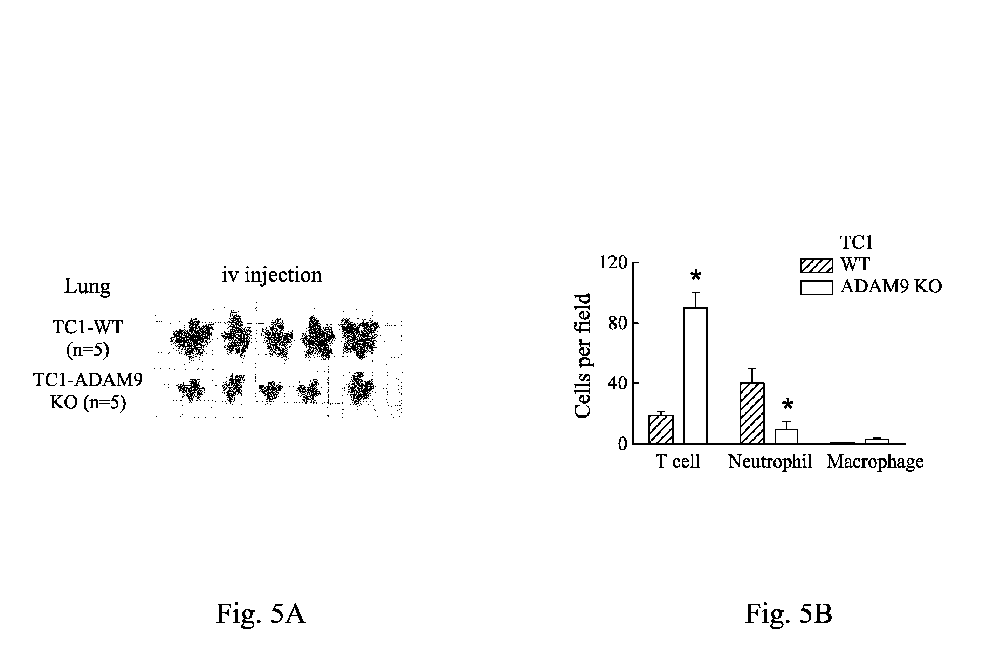

Please refer to Figs. 5A and 5B, which show the analysis results of the effect of ADAM9 knockout on lung metastatic tumor growth and immune cell infiltration. Fig. 5B shows immunohistochemistry of inflammatory cells in lung tissue with antibodies against T cells (CD3), macrophages (F4/80) and neutrophils (MPO) to detect the expression of T cells, macrophages and neutrophils in tumor tissues of TC1-WT group and TC1-ADAM9 KO group. The results of Figs. 5A and 5B show that TC1-ADAM9 KO lung cancer cells reduce the size of lung metastatic tumors. In addition, increased CD8⁺ T cell infiltration can be detected in the tumors of mice in TC1-ADAM9 KO group, while the number of neutrophils is reduced.

From the foregoing results, it can be seen that the ADAM9 gene has an impact on tumor growth and immune cell infiltration. This experiment further conducted a comprehensive analysis of genes involved in ADAM9-mediated immunosuppression via RNA sequencing of lung tumors, and revealed that ADAM9 in cancer cells might influence and interact with immune cells in the microenvironment, thereby contributing to tumor development, progression, and metastasis. To precisely identify the ADAM9-regulated genes, we identified the genes whose expression profiles are concordant in cancer cell lines and in subcutaneous tumors from TC1-WT group and TC1-ADAM9 KO group of mice as ADAM9-mediated tumor genes (329 genes). Please refer to Table 6, which shows the functional analysis of 329 ADAM9-mediated tumor genes influenced in cultured cells and tumors.

**Table 6**

| Function | Gene number | -log (*p*-value) |
|---|---|---|
| Regulation of cytokine production | 18 | 6.1 |
| Positive regulation of multicellular organismal process | 20 | 5.3 |
| Negative regulation of multicellular organismal process | 15 | 5.2 |
| Response to IFN-β | 5 | 4.8 |
| Response to IFN-γ | 6 | 4.6 |
| Leukocyte tethering or rolling | 4 | 4.6 |
| Negative regulation of immune system | 11 | 4.5 |
| Defense response to virus | 9 | 4.4 |

From this analysis, it can be seen that ADAM9-mediated tumor genes function in the regulation of IFN-γ and cytokine that produce and respond to IFN-γ. Therefore, in the experiment, the target cells of C57BL/6 mice (TC1-WT lung cancer cells or TC1-ADAM9 KO lung cancer cells) and effector cells (spleen cells of C57BL/6 mice) were co-cultured at a ratio of 1:40 for 24 hours. The cell apoptosis rate was measured to verify the analysis results.

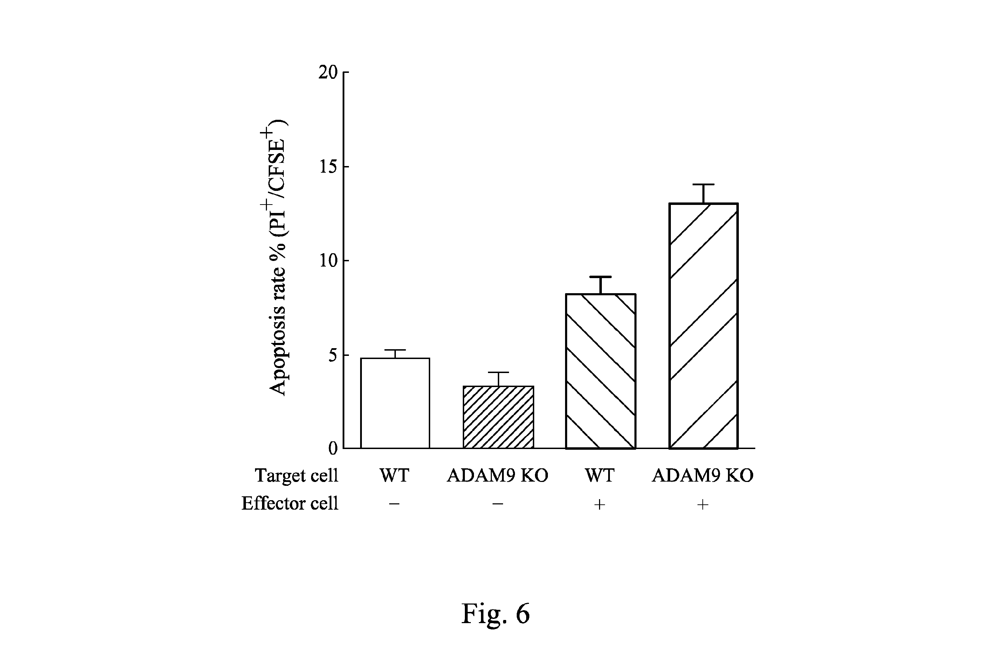

Please refer to Fig. 6, which shows the analysis results of the apoptosis rate of target cells and effector cells after 24 hours of co-cultivation, wherein WT represents TC1-WT lung cancer cells and ADAM9 KO represents TC1-ADAM9 KO lung cancer cells. The results show that TC1-WT lung cancer cells are more resistant to immune cell attack than TC1-ADAM9 KO lung cancer cells. These results indicate that the ADAM9 knockout in tumors can increase the infiltration of immune cells and be more sensitive to immune cell responses.

In order to further evaluate whether the treatment of ADAM9 inhibitors will give the same results as the ADAM9 gene knockout, TC1-WT lung cancer cells were injected subcutaneously into C57BL/6 mice with normal immunity to establish a subcutaneous lung tumor mouse model, and the ADAM9 inhibitor (the compound SW1, 10 mg/kg) was treated by subcutaneous injection. From Day 15 to Day 24, the compound SW1 was injected once a day for 9 days. The tumor volume was observed on Day 12, Day 20 and Day 27, respectively.

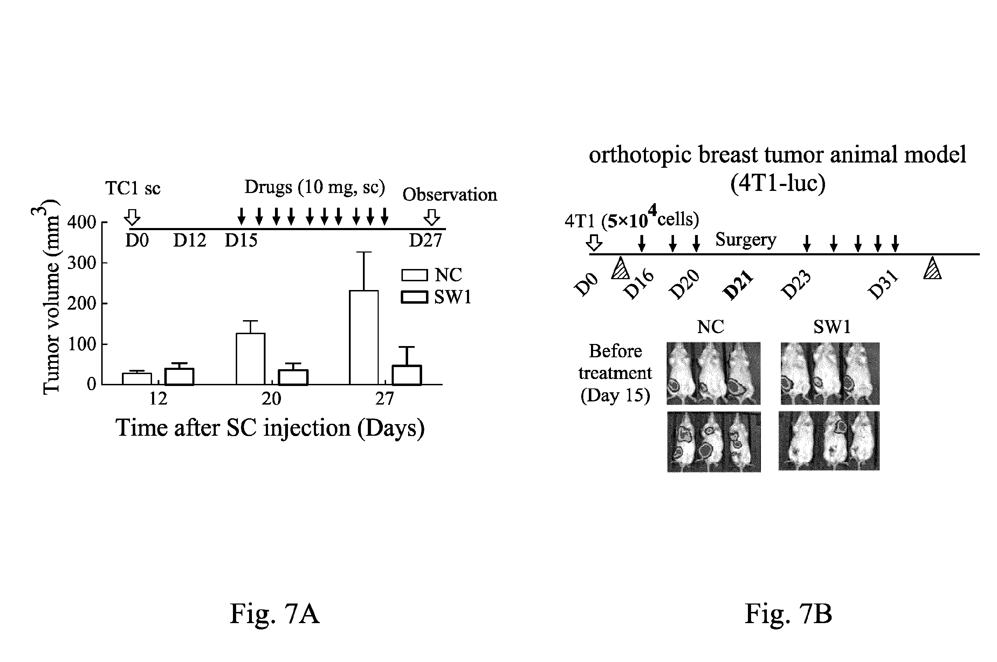

Please refer to Fig. 7A, which shows the analysis results of the anti-tumor effect of the ADAM9 inhibitor. The control group was injected with DMSO as a control for the treatment of the ADAM9 inhibitor. The results of Fig. 7A show that, compared with the control group treated with DMSO, the treatment of the compound SW1 reduces tumor growth in mice with subcutaneous lung tumors (N = 5 per group).

Please further refer to Fig. 7B, which is a flow chart of the ADAM9 inhibitor used in the treatment of a syngeneic orthotopic breast tumor animal model. In the experiment, 4T1-luc breast cancer cells (5×10⁴) were transplanted into the breast fat pads of BALB/c mice to establish the syngeneic orthotopic breast tumor animal model. The IVIS imaging system is used for detection, and the tumor size is represented by the luminous flux of the IVIS image. After tumor images were detected on Day 15, the ADAM9 inhibitor (the compound SW1, 10 mg/kg) was pretreated by subcutaneous injection on Day 16, Day 18, and Day 20. After the tumor was surgically removed on Day 21, the ADAM9 inhibitor (the compound SW1, 10 mg/kg) was continuously treated on Day 23, Day 25, Day 27, Day 29 and Day 31. The surgically resected syngeneic orthotopic breast tumor animal model tumors were stained by immunohistochemistry. In the experiment, a control group was injected with DMSO as a control for the treatment of the ADAM9 inhibitor.

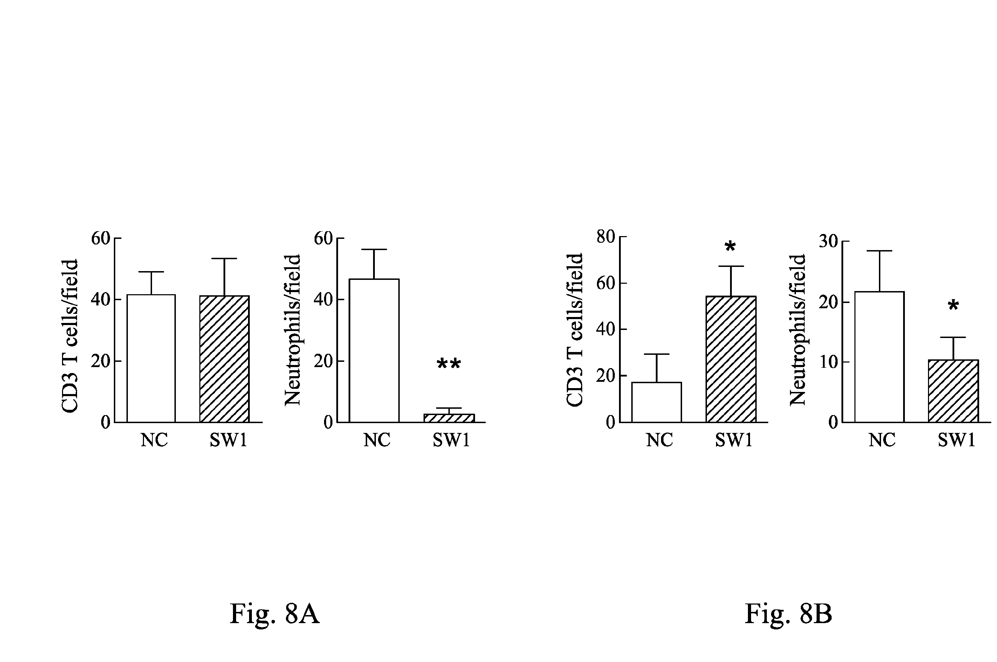

Please further refer to Figs. 8A and 8B, which show the analysis results of the effect of the ADAM9 inhibitor on immune cell infiltration. Fig. 8A shows the analysis results of calculated infiltrating T cells and neutrophils in primary 4T1 breast tumors, and Fig. 8B shows the analysis results of calculated infiltrating T cells and neutrophils in lung metastatic breast tumors. The results of Figs. 8A and 8B show that the number of infiltrating T cells was increased in 4T1 breast tumors treated with the ADAM9 inhibitor (the compound SW1), especially in the lung metastatic breast tumors. In 4T1 primary breast tumors and lung metastatic breast tumors, the number of infiltrating neutrophils was greatly reduced. The results indicate that the ADAM9 inhibitor can increase infiltrating T cells in 4T1 breast tumors and reduce infiltrating neutrophils.

To further analyze whether the ADAM9 inhibitor and the checkpoint inhibitor have a synergistic effect, in the experiment, TC1 subcutaneous lung tumor mice were treated alone with the ADAM9 inhibitor (the compound SW1), the checkpoint inhibitor (anti-PDL1 antibody) or co-treated with the ADAM9 inhibitor (the compound SW1) and the checkpoint inhibitor (anti-PDL1 antibody), and the ratio of CD8⁺ T cells and regulatory T cells (T_{Reg}) in tumors of TC1 subcutaneous lung tumor mice was analyzed. The experiment also included a control group treated with vehicle (DMSO).

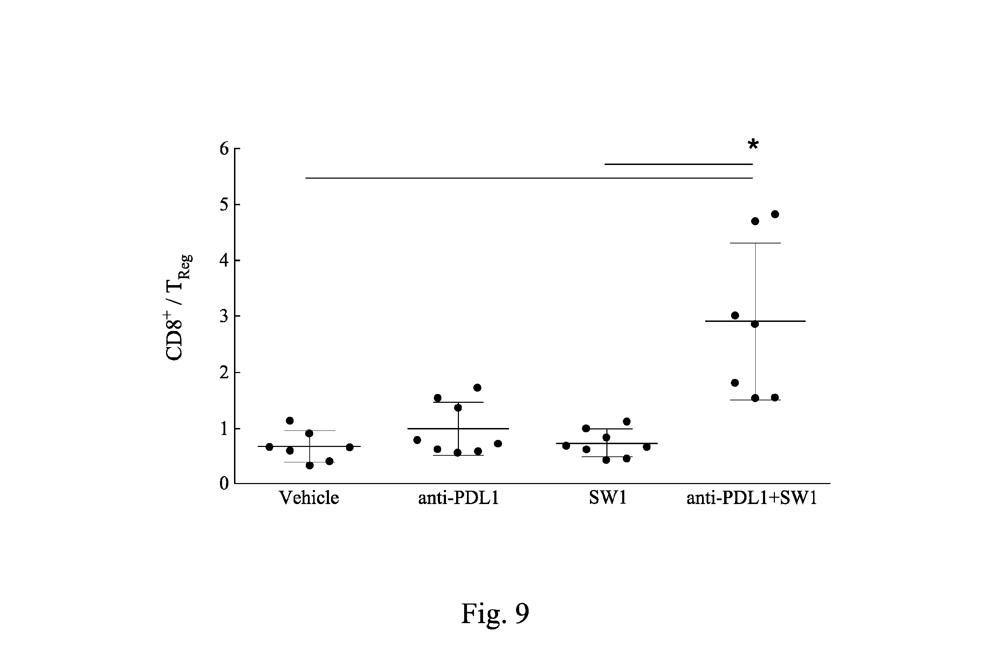

Please refer to Fig. 9, which shows the analysis results of co-treatment of the ADAM9 inhibitor and the checkpoint inhibitor. Compared with the group treated with the vehicle, the compound SW1 alone or anti-PDL1 antibody alone, the ratio of CD8⁺ T cells to regulatory T cells (T_{Reg}) increased significantly in the group co-treated with the compound SW1 and anti-PDL1 antibody. The results indicate that the ADAM9 inhibitor and the checkpoint inhibitor have synergistic effect.

We further analyzed whether the treatment of the ADAM9 inhibitor can stimulate tumor cells to secrete cytokines to activate the anti-tumor immunity of the ADAM9 inhibitor. In the experiment, human colon cancer cells SW620 and human pancreatic cancer cells PANC-1 were treated with 12.5 µM of the compound SW1, and human esophageal cancer cells CE146T were treated with 5 µM of the compound SW1. After 24 hours, the cells were collected and the RNA expression levels of CXCL10, CXCL11, CXC3CL1 and IFNB were detected by RT-qPCR. CXCL10, CXCL11 and CXCL1 are cytokines that promote the immune response of CD8⁺ T cells, and IFNB is a cytokine with anti-cancer effects.

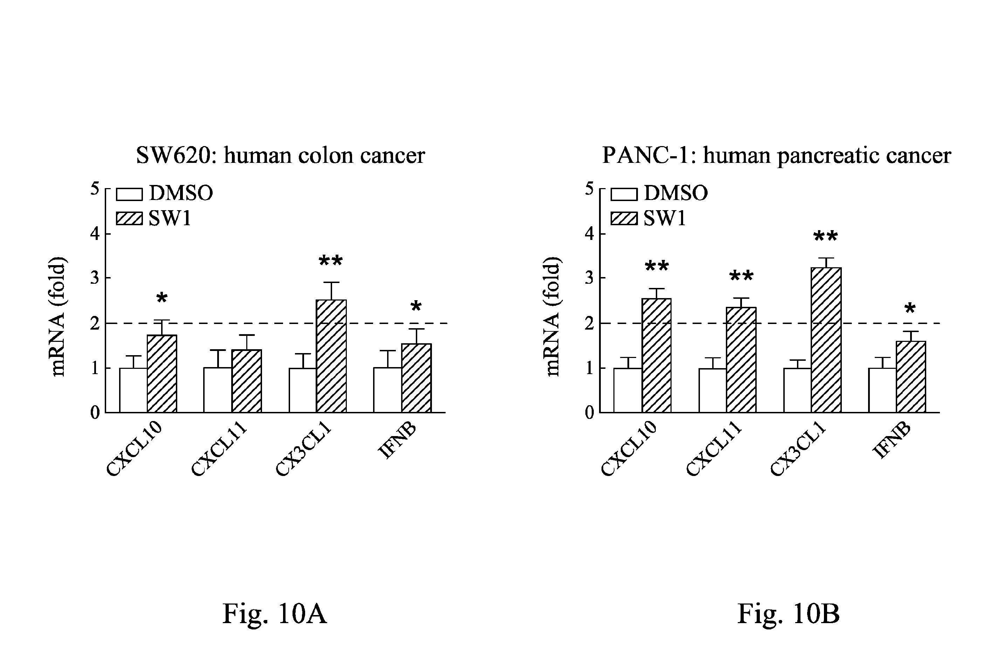

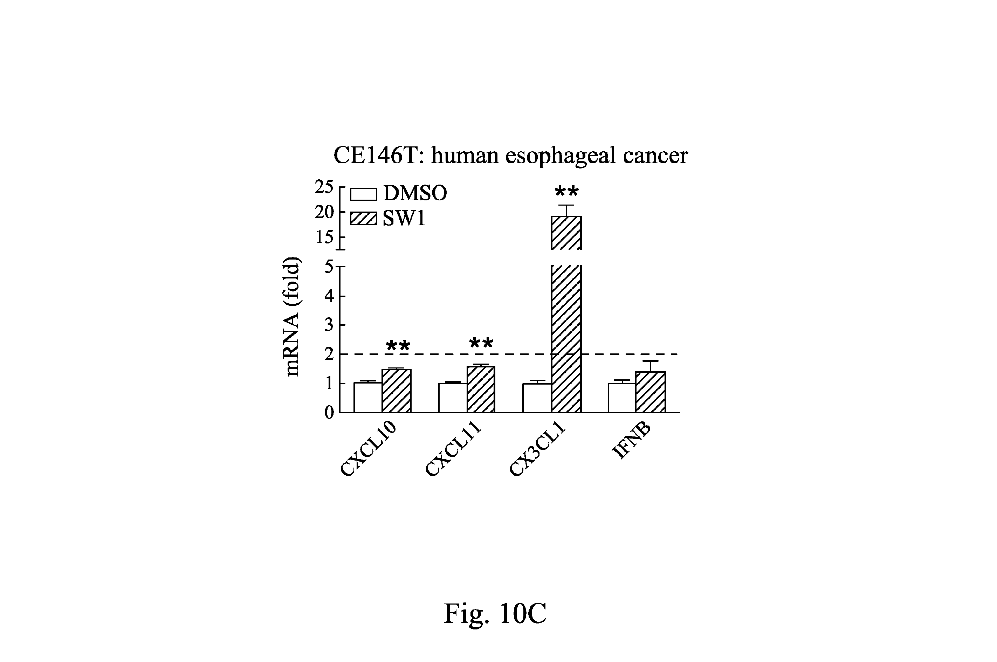

Please refer to Figs. 10A, 10B and 10C, which show the analysis results of the ADAM9 inhibitor stimulated tumor cells to secrete cytokines. The results of Figs. 10A to 10C show that treatment of the compound SW1 can increase the expressions of CXCL10, CXCL11, CXC3CL1 and IFNB in human colon cancer cells, human pancreatic cancer cells and human esophageal cancer cells, indicating that the ADAM9 inhibitor can stimulate cancer cells to secrete cytokines related to immunity activation.

In summary, the present disclosure provides a novel use of the ADAM9 inhibitor as defined herein, which can be used as an immunomodulator to enhance the effectiveness of cancer immunotherapy. Experimental data confirms that the ADAM9 inhibitor can reduce tumor growth, modify the tumor microenvironment, stimulate immune cells to infiltrate the tumor, stimulate cancer cells to secrete cytokines related to immune activation; thereby the ADAM9 inhibitor can be used to treat the immunotherapeutic-resistant tumor. Therefore, the ADAM9 inhibitor can be used in cancer immunotherapy and can treat cancer, and the ADAM9 inhibitor has synergistic effect when formulated with the checkpoint inhibitor. The ADAM9 inhibitor can also increase the effectiveness of cancer immunotherapy, and has the potential to be used in the biomedical and health care market.

## Claims

1. An ADAM9 inhibitor for use in being an immunomodulator, wherein the ADAM9 inhibitor is a compound of Formula (I), or a pharmaceutically acceptable salt or stereoisomer thereof, 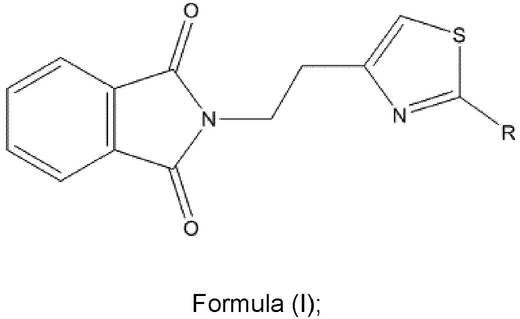 wherein:
R is a C₁-C₁₈ hydrocarbyl comprising 0-3 heteroatoms independently selected from N, S and O, or a C₁-C₁₈ hydrocarbyl comprising 0-3 heteroatoms independently selected from N, S and O substituted with a heteroatom selected from N, S or O; and
R comprises a C₅-C₁₀ organocyclic, and the organocyclic is an aryl comprising 0-3 heteroatoms independently selected from N, S and O.

2. The ADAM9 inhibitor for use of claim 1, wherein the immunomodulator is used to enhance an effectiveness of a cancer immunotherapy.

3. The ADAM9 inhibitor for use of claims 1 or 2, wherein a subject of the cancer immunotherapy is an immune-compromised patient.

4. The ADAM9 inhibitor for use of any one of claims 1 to 3, wherein the immunomodulator is used to stimulate an infiltration of immune cells into a tumor.

5. The ADAM9 inhibitor for use of claim 4, wherein the immune cells are T cells, natural killer cells, macrophages, neutrophils, dendritic cells or suppressor cells derived from bone marrow.

6. The ADAM9 inhibitor for use of any one of claims 1 to 5, wherein the immunomodulator is used to modify a chemokine profile of a tumor microenvironment.

7. The ADAM9 inhibitor for use of any one of claims 1 to 6, wherein the immunomodulator is used to treat an immunotherapeutic-resistant tumor.

8. The ADAM9 inhibitor for use of claim 7, wherein the immunotherapeutic-resistant tumor is resistant to a checkpoint inhibitor, an adoptive cell transfer, a therapeutic antibody, a treatment vaccine, a cytokine, an immune cell therapy or a combination thereof.

9. The ADAM9 inhibitor for use of claim 8, wherein the checkpoint inhibitor is an anti-CTLA-4 antibody, an anti-PDL1 antibody or an anti-PD-1 antibody.

10. The ADAM9 inhibitor for use of any one of claims 1 to 9, wherein the immunomodulator is formulated with a checkpoint inhibitor.

11. The ADAM9 inhibitor for use of claim 10, wherein the checkpoint inhibitor is an anti-CTLA-4 antibody, an anti-PDL1 antibody or an anti-PD-1 antibody.

12. The ADAM9 inhibitor for use of claim 1, wherein R is NHR₁, and R₁ is 2-thiazol-4-yl-ethyl-isoindoline-1,3-dione.

## Patentansprüche

1. ADAM9-Inhibitor zur Verwendung als Immunmodulator, wobei der ADAM9-Inhibitor eine Verbindung der Formel (I) oder ein pharmazeutisch unbedenkliches Salz oder Stereoisomer davon ist, 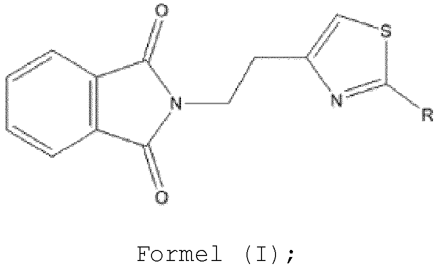 wobei:
R für C₁-C₁₈-Hydrocarbyl mit 0-3 unabhängig voneinander aus N, S und O ausgewählten Heteroatomen oder C₁-C₁₈-Hydrocarbyl mit 0-3 unabhängig voneinander aus N, S und O ausgewählten Heteroatomen und substituiert mit einem aus N, S oder O ausgewählten Heteroatom steht und
R einen C₅-C₁₀-Organocyclus umfasst und der Organocyclus für Aryl mit 0-3 unabhängig voneinander aus N, S und O ausgewählten Heteroatomen steht.

2. ADAM9-Inhibitor zur Verwendung nach Anspruch 1, wobei der Immunmodulator verwendet wird, um eine Wirksamkeit einer Krebsimmuntherapie zu erhöhen.

3. ADAM9-Inhibitor zur Verwendung nach Anspruch 1 oder 2, wobei ein Subjekt der Krebsimmuntherapie ein immungeschwächter Patient ist.

4. ADAM9-Inhibitor zur Verwendung nach einem der Ansprüche 1 bis 3, wobei der Immunmodulator verwendet wird, um eine Infiltration von Immunzellen in einen Tumor zu stimulieren.

5. ADAM9-Inhibitor zur Verwendung nach Anspruch 4, wobei es sich bei den Immunzellen um T-Zellen, natürliche Killerzellen, Makrophagen, neutrophile Zellen, dendritische Zellen oder Suppressorzellen, die aus Knochenmark stammen, handelt.

6. ADAM9-Inhibitor zur Verwendung nach einem der Ansprüche 1 bis 5, wobei der Immunmodulator verwendet wird, um ein Chemokinprofil einer Tumormikroumgebung zu modifizieren.

7. ADAM9-Inhibitor zur Verwendung nach einem der Ansprüche 1 bis 6, wobei der Immunmodulator zur Behandlung eines immuntherapeutisch resistenten Tumors verwendet wird.

8. ADAM9-Inhibitor zur Verwendung nach Anspruch 7, wobei der immuntherapeutisch resistente Tumor gegenüber einem Checkpoint-Inhibitor, einem adoptiven Zelltransfer, einem therapeutischen Antikörper, einem Behandlungsimpfstoff, einem Zytokin, einer Immunzelltherapie oder einer Kombination davon resistent ist.

9. ADAM9-Inhibitor zur Verwendung nach Anspruch 8, wobei der Checkpoint-Inhibitor ein Anti-CTLA-4-Antikörper, ein Anti-PDL1-Antikörper oder ein Anti-PD-1-Antikörper ist.

10. ADAM9-Inhibitor zur Verwendung nach einem der Ansprüche 1 bis 9, wobei der Immunmodulator mit einem Checkpoint-Inhibitor formuliert ist.

11. ADAM9-Inhibitor zur Verwendung nach Anspruch 10, wobei der Checkpoint-Inhibitor ein Anti-CTLA-4-Antikörper, ein Anti-PDL1-Antikörper oder ein Anti-PD-1-Antikörper ist.

12. ADAM9-Inhibitor zur Verwendung nach Anspruch 1, wobei R für NHR₁ steht und R₁ für 2-Thiazol-4-ylethylisoindolin-1,3-dion steht.

## Revendications

1. Inhibiteur d'ADAM9 pour une utilisation comme immunomodulateur, dans lequel l'inhibiteur d'ADAM9 est un composé de formule (I), ou un sel ou stéréoisomère pharmaceutiquement acceptable de celui-ci, 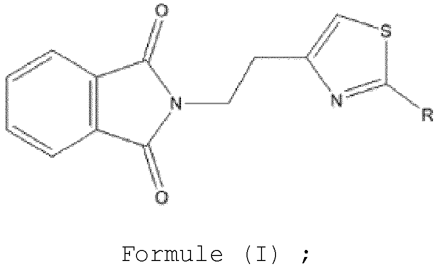 dans laquelle :
R est un hydrocarbyle en C₁-C₁₈ comprenant 0-3 hétéroatomes indépendamment choisis parmi N, S et O, ou un hydrocarbyle en C₁-C₁₈ comprenant 0-3 hétéroatomes indépendamment choisis parmi N, S et O substitué par un hétéroatome choisi parmi N, S ou O ; et
R comprend un organocyclique en C₅-C₁₀, et l'organocyclique est un aryle comprenant 0-3 hétéroatomes choisis indépendamment parmi N, S et O.

2. Inhibiteur d'ADAM9 pour une utilisation selon la revendication 1, dans lequel l'immunomodulateur est utilisé pour augmenter l'efficacité d'une immunothérapie du cancer.

3. Inhibiteur d'ADAM9 pour une utilisation selon la revendication 1 ou 2, dans lequel un sujet de l'immunothérapie du cancer est un patient immunodéprimé.

4. Inhibiteur d'ADAM9 pour une utilisation selon l'une quelconque des revendications 1 à 3, dans lequel l'immunomodulateur est utilisé pour stimuler une infiltration de cellules immunitaires dans une tumeur.

5. Inhibiteur d'ADAM9 pour une utilisation selon la revendication 4, dans lequel les cellules immunitaires sont des cellules T, des cellules tueuses naturelles, des macrophages, des neutrophiles, des cellules dendritiques ou des cellules suppressives dérivées de la moelle osseuse.

6. Inhibiteur d'ADAM9 pour une utilisation selon l'une quelconque des revendications 1 à 5, dans lequel l'immunomodulateur est utilisé pour modifier un profil de chimiokine d'un microenvironnement tumoral.

7. Inhibiteur d'ADAM9 pour une utilisation selon l'une quelconque des revendications 1 à 6, dans lequel l'immunomodulateur est utilisé pour traiter une tumeur résistante à une immunothérapie.

8. Inhibiteur d'ADAM9 pour une utilisation selon la revendication 7, dans lequel la tumeur résistante à une immunothérapie est résistante à un inhibiteur de point de contrôle, à un transfert de cellules adoptives, à un anticorps thérapeutique, à un vaccin de traitement, à une cytokine, à une thérapie cellulaire immunitaire ou à une combinaison de ceux-ci.

9. Inhibiteur d'ADAM9 pour une utilisation selon la revendication 8, dans lequel l'inhibiteur de point de contrôle est un anticorps anti-CTLA-4, un anticorps anti-PDL1 ou un anticorps anti-PD-1.

10. Inhibiteur d'ADAM9 pour une utilisation selon l'une quelconque des revendications 1 à 9, dans lequel l'immunomodulateur est formulé avec un inhibiteur de point de contrôle.

11. Inhibiteur d'ADAM9 pour une utilisation selon la revendication 10, dans lequel l'inhibiteur de point de contrôle est un anticorps anti-CTLA-4, un anticorps anti-PDL1 ou un anticorps anti-PD-1.

12. Inhibiteur d'ADAM9 pour une utilisation selon la revendication 1, dans lequel R est NHR₁, et R₁ est 2-thiazol-4-yl-éthyl-isoindoline-1,3-dione.
